(19) Europäisches Patentamt
European Patent Office
Office européen des brevets

(11) EP 0 994 692 B1

(12) **FASCICULE DE BREVET EUROPEEN**

(45) Date de publication et mention de la délivrance du brevet:
**23.04.2003 Bulletin 2003/17**

(21) Numéro de dépôt: **99907684.7**

(22) Date de dépôt: **11.03.1999**

(51) Int Cl.$^7$: **A61K 7/13**

(86) Numéro de dépôt international:
**PCT/FR99/00541**

(87) Numéro de publication internationale:
**WO 99/048465 (30.09.1999 Gazette 1999/39)**

(54) **COMPOSITION DE TEINTURE D'OXYDATION DES FIBRES KERATINIQUES CONTENANT UN DERIVE AZO DE 3-AMINOPYRIDINE ET PROCEDE DE TEINTURE METTANT EN OEUVRE CETTE COMPOSITION**

OXIDATIONSFÄRBEZUSAMMENSETZUNG FÜR KERATINISCHE FASERN, DIE AZODERIVATE VON 3-AMINO-PYRIDIN ENTHÄLT, UND FÄRBEVERFAHREN

OXIDATION DYEING COMPOSITION FOR KERATINOUS FIBRES CONTAINING A 3-AMINOPYRIDINE AZO DERIVATIVE AND DYEING METHOD USING SAID COMPOSITION

(84) Etats contractants désignés:
**AT BE CH CY DE DK ES FI FR GB GR IE IT LI LU MC NL PT SE**

(30) Priorité: **20.03.1998 FR 9803454**

(43) Date de publication de la demande:
**26.04.2000 Bulletin 2000/17**

(73) Titulaire: **L'OREAL**
**75008 Paris (FR)**

(72) Inventeurs:
- **LANG, Gérard**
  **F-95390 Saint Prix (FR)**
- **COTTERET, Jean**
  **F-78480 Verneuil-sur-Seine (FR)**
- **MAUBRU, Mireille**
  **F-78400 Chatou (FR)**

(74) Mandataire: **Goulard, Sophie et al**
**L'OREAL,**
**DPI,**
**6, rue Sincholle**
**92585 Clichy Cedex (FR)**

(56) Documents cités:
**EP-A- 0 739 622**
**EP-A- 0 850 638**
**WO-A-97/39727**
**DE-A- 4 241 173**
**US-A- 4 025 301**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

L'invention a pour objet une composition pour la teinture d'oxydation des fibres kératiniques, et en particulier des fibres kératiniques humaines telles que les cheveux comprenant, dans un milieu approprié pour la teinture, au moins une base d'oxydation hétérocyclique, et au moins un dérivé de l'amino-3 pyridine à titre de colorant direct, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

Il est également connu que pour faire encore varier les nuances obtenues et leur donner des reflets, on peut utiliser, en association avec les précurseurs de colorants d'oxydation et les coupleurs, des colorants directs, c'est à dire des substances colorées qui apportent une coloration en l'absence d'agent oxydant.

Ces colorants directs appartiennent pour leur très grande majorité à la famille des composés nitrés de la série benzénique et ont l'inconvénient, lorsqu'ils sont incorporés dans des compositions tinctoriales, de conduire à des colorations présentant une ténacité insuffisante, en particulier vis-à-vis des shampooings.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans la demande de brevet FR-A-2 285 851, des compositions pour la teinture d'oxydation des fibres kératiniques contenant l'association d'une base d'oxydation benzénique et d'un colorant direct de la famille des 3-amino pyridines. Cependant les colorations obtenues en mettant en oeuvre de telles compositions ne sont pas entièrement satisfaisantes, notamment du point de vue de leur chromaticité et de leur ténacité.

Or, la demanderesse vient maintenant de découvrir qu'il est possible d'obtenir de nouvelles teintures, capables de conduire à des colorations puissantes et chromatiques, peu sélectives et résistant bien aux diverses agressions que peuvent subir les fibres, en associant au moins une base d'oxydation hétérocyclique, et au moins un dérivé de 3-amino pyridine convenablement sélectionné à titre de colorant direct,.

Cette découverte est à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :

- au moins une base d'oxydation hétérocyclique, et
- à titre de colorant direct, au moins un dérivé de 3-amino pyridine choisi parmi les composés de formule (I) suivante :

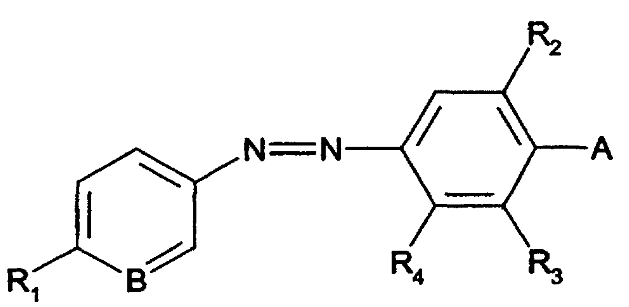

(I)

dans laquelle :

- B représente un groupement de formules (Ia) ou (Ib) suivantes :

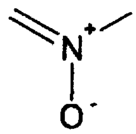 (Ia) 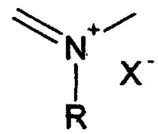 (Ib)

- R représente un radical alkyle en $C_1$-$C_4$ ;
- $R_1$ représente un atome d'hydrogène ou d'halogène tel que le chlore, le brome ou le fluor, un radical alkyle en $C_1$-$C_4$ ou alcoxy en $C_1$-$C_4$ ;
- $R_2$ représente un atome d'hydrogène, un radical alkyle en $C_1$-$C_4$ ou alcoxy en $C_1$-$C_4$ ;
- $R_4$ représente un atome d'hydrogène ou d'halogène tel que le chlore, le brome ou le fluor, un radical alkyle en $C_1$-$C_4$, nitro, amino ou acyl($C_1$-$C_4$)amino ;
- $R_3$ représente un atome d'hydrogène ou bien $R_4$ et $R_3$ forment un cycle insaturé à 6 chaînons portant un substituant hydroxyle chélaté avec un des atomes d'azote de la double liaison azoïque :
- A représente un reste $-NR_5R_6$ dans lequel $R_5$ représente un atome d'hydrogène, un radical alkyle en $C_1$-$C_4$, monohydroxyalkyle en $C_1$-$C_4$ ou polyhydroxyalkyle en $C_2$-$C_4$; $R_6$ représente un atome d'hydrogène, un radical alkyle en $C_1$-$C_4$, monohydroxyalkyle en $C_1$-$C_4$ ou polyhydroxyalkyle en $C_2$-$C_4$, un cycle phényle ou un radical $-CH_2-SO_3Na$ ;
- $X^-$ représente un anion monovalent ou divalent et est de préférence choisi parmi un atome d'halogène tel que le chlore, le brome, le fluor ou l'iode, un hydroxyde, un hydrogènesulfate, ou un alkyl($C_1$-$C_6$)sulfate tel que par exemple un méthylsulfate ou un éthylsulfate.

La composition tinctoriale conforme à l'invention conduit à des colorations puissantes, chromatiques, présentant une faible sélectivité et d'excellentes propriétés de résistances à la fois vis à vis des agents atmosphériques tels que la lumière et les intempéries et vis à vis de la transpiration et des différents traitements que peuvent subir les cheveux. Ces propriétés sont particulièrement remarquables en ce qui concerne la chromaticité.

L'invention a également pour objet un procédé de teinture d'oxydation des fibres kératiniques mettant en oeuvre cette composition tinctoriale.

La ou les bases d'oxydation hétérocycliques sont de préférence choisies parmi les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-333 495, comme la 2,4,5,6-tétra-aminopyrimidine, la 4-hydroxy 2,5,6-triaminopyrimidine, et leurs sels d'addition avec un acide, ainsi que les dérivés pyrazolopyrimidiniques tels que la pyrazolo-[1,5-a]-pyrimidine-3,7-diamine, la 2-méthyl pyrazolo-[1,5-a]-pyrimidine-3,7-diamine, la 2,5-diméthyl pyrazolo-[1,5-a]-pyrimidine-3,7-diamine, la pyrazolo-[1,5-a]-pyrimidine-3,5-diamine, la 2,7-diméthyl pyrazolo-[1,5-a]-pyrimidine-3,5-diamine, le 3-amino pyrazolo-[1,5-a]-pyrimidin-7-ol, le 3-amino 5-méthyl pyrazolo-[1,5-a]-pyrimidin-7-ol, le 3-amino pyrazolo-[1,5-a]-pyrimidin-5-ol, le 2-(3-amino pyrazolo-[1,5-a]-pyrimidin-7-ylamino)-éthanol, la 3-amino-7-β-hydroxyéthylamino-5-méthyl-pyrazolo-[1,5-a]-pyrimidine, le 2-(7-amino pyrazolo-[1,5-a]-pyrimidin-3-ylamino)-éthanol, le 2-[(3-amino-pyrazolo-[1,5-a]-pyrimidin-7-yl)-(2-hydroxyéthyl)-amino]-éthanol, le 2-[(7-amino-pyrazolo-[1,5-a]-pyrimidin-3-yl)-(2-hydroxyéthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N-7, N-7-tetraméthyl pyrazolo-[1,5-a]-pyrimidine-3,7-diamine, et leurs sels d'addition et leurs formes tautomères, lorsqu'il existe un équilibre tautomérique.

Parmi les dérivés pyrazoliques, on peut plus particulièrement citer les composés décrits dans les brevets ou demandes de brevet DE 3 843 892, DE 4 133 957, DE 4 234 886, DE 4 234 887, FR 2 733 749, FR 2 735 685, WO 94/08969 et WO 94/08970, comme le 4,5-diamino pyrazole, le 4,5-diamino 1-méthyl pyrazole, le 1-benzyl 4,5-diamino pyrazole, le 3,4-diamino pyrazole, le 1-benzyl 4,5-diamino 3-méthyl pyrazole, le 4-amino 1,3-diméthyl 5-hydrazino pyrazole, le 4,5-diamino 3-méthyl 1-phényl pyrazole, le 4,5-diamino 3-méthyl 1-tert-butyl pyrazole, le 4,5-diamino 1-méthyl 3-tert-butyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, et leurs sels d'addition avec un acide.

La ou les bases d'oxydation hétérocycliques représentent de préférence de 0,0005 à 12 % en poids environ du poids total de la composition tinctoriale conforme à l'invention, et encore plus préférentiellement de 0,005 à 6 % en poids environ de ce poids.

Le ou les dérivés de 3-amino pyridine de formule (I) conformes à l'invention sont de préférence choisis parmi :

- le méthosulfate de diméthylamino-4' benzène azo-1' : 3-méthyl-1-pyridinium de formule :

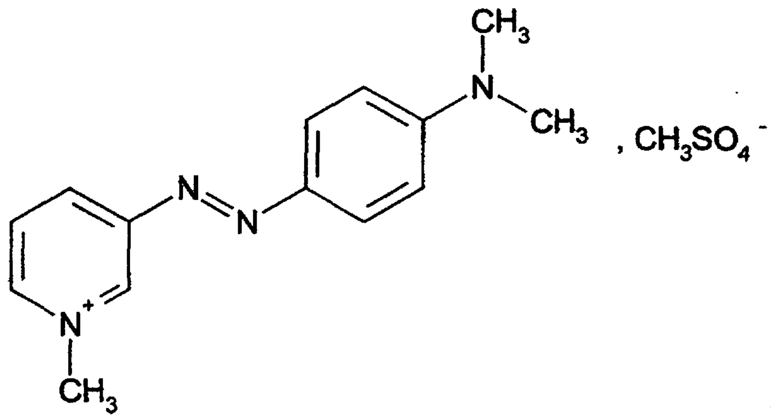

;

- le méthosulfate de bis-(β-hydroxyéthyl)amino-4'-benzène-azo-1' : 3-méthyl-1-pyridinium de formule :

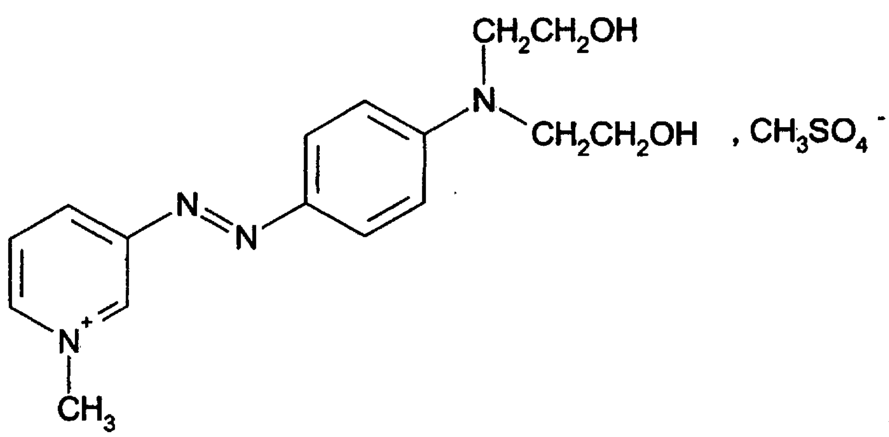

;

- le méthosulfate d'amino-4'-hydroxy-8'-naphtalène-azo-1' : 3-méthyl-1-pyridinium de formule

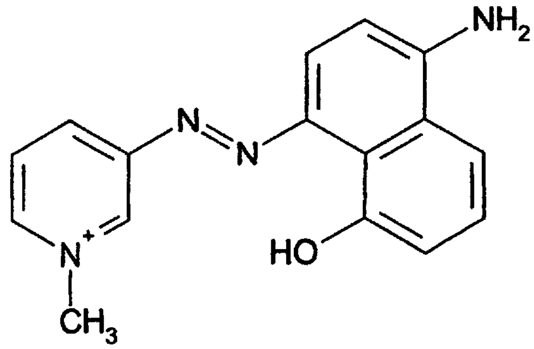

, $CH_3SO_4^-$

;

- le méthosulfate de diméthylamino-4'-nitro-2'-benzène-azo-1' : 3-méthyl-1-pyridinium de formule :

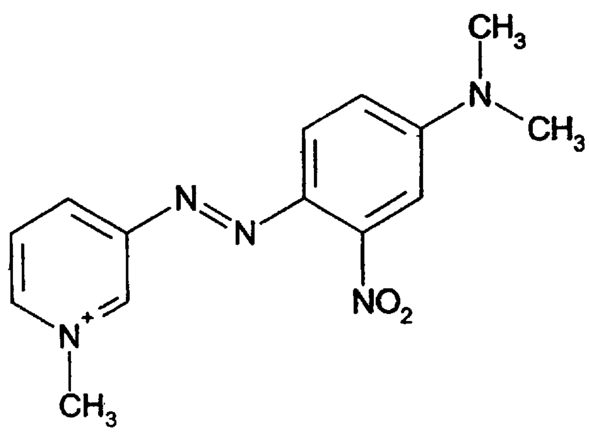

, $CH_3SO_4^-$

;

- le méthosulfate de diméthylamino-4'-benzène-azo-1' : 3-diméthyl-1,6-pyridinium de formule

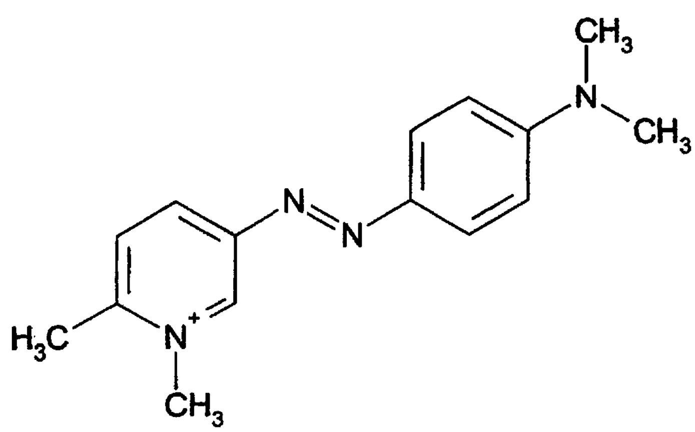

, $CH_3SO_4^-$

;

- l'amino-4'-benzène-azo-1' : 3-pyridine N-oxyde de formule :

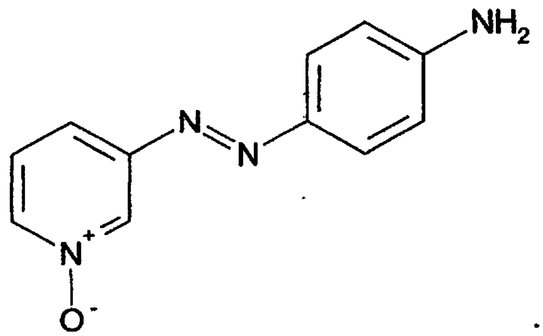

;

- la diméthylamino4'-benzène-azo-1' : 3-pyridine N-oxyde de formule

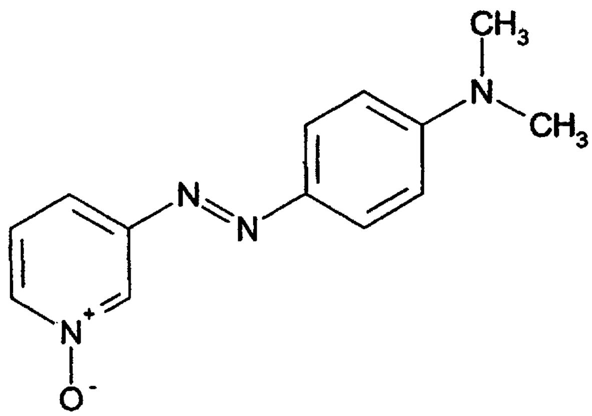

;

- la N,N-bis-(β-hydroxyéthyl)amino-4'-benzène-azo-1' : 3-pyridine N-oxyde de formule :

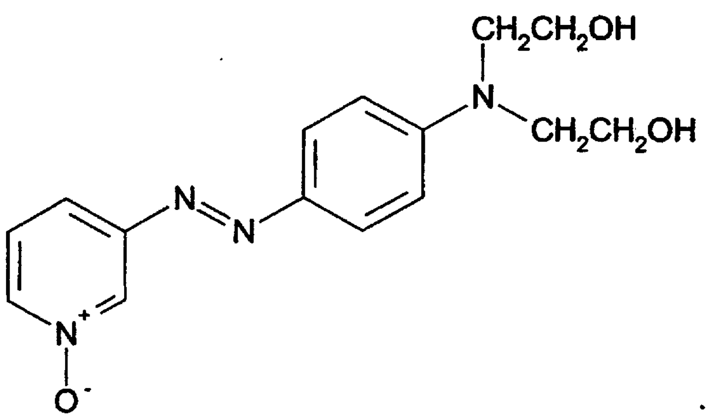

;

- l'éthosulfate de diméthy(amino-4'-méthyl-2'-benzène-azo-1' : 3-éthyl-1-pyridinium de formule :

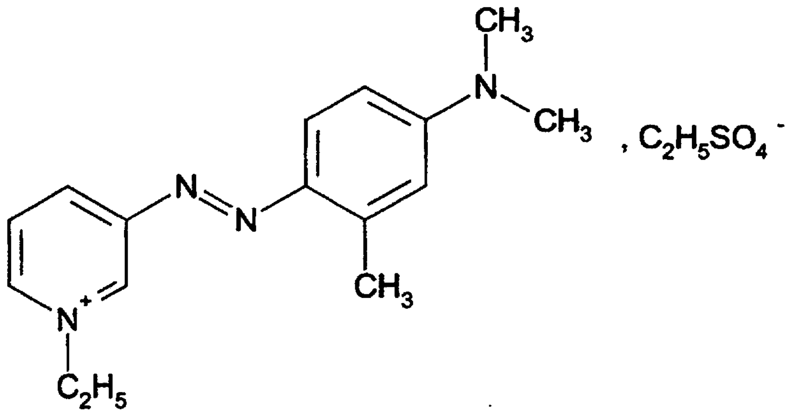

;

- le bromure de diméthylamino-4'-méthyl-2'-benzène-azo-1' : 3-butyl-1-pyridinium de formule

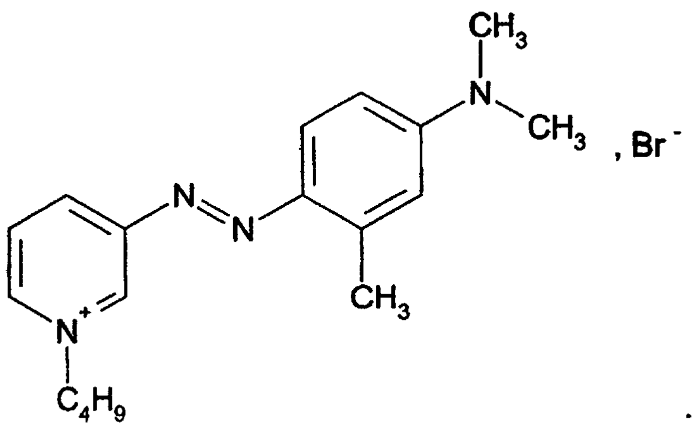

;

- le méthosulfate de diméthylamino-4'-chloro-2'-benzène-azo-1' : 3-méthyl-1-pyridinium de formule :

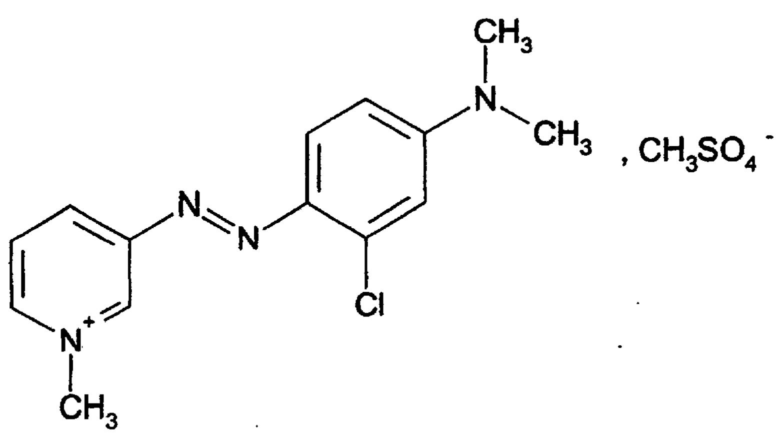

;

- le méthosulfate de diamino-2',4'-méthyl-5'-benzène-azo-1' : 3-méthyl-1 pyridinium de formule :

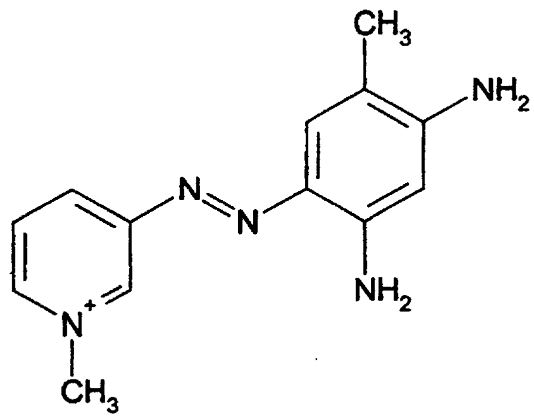

, $CH_3SO_4^-$

;

- le méthosulfate de phénylamino-4'-benzène-azo-1': 3-méthyl-1-pyridinum de formule

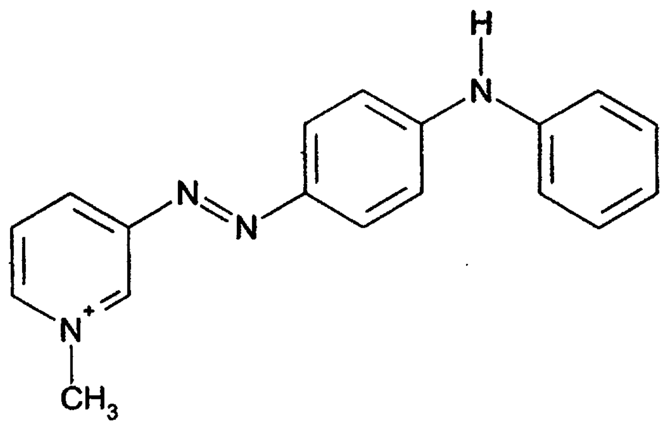

, $CH_3SO_4^-$

;

- l'éthosulfate d'acétylamino-2'-diméthylamino-4'-benzène-azo-1' : 3-éthyl-1-pyridinium de formule :

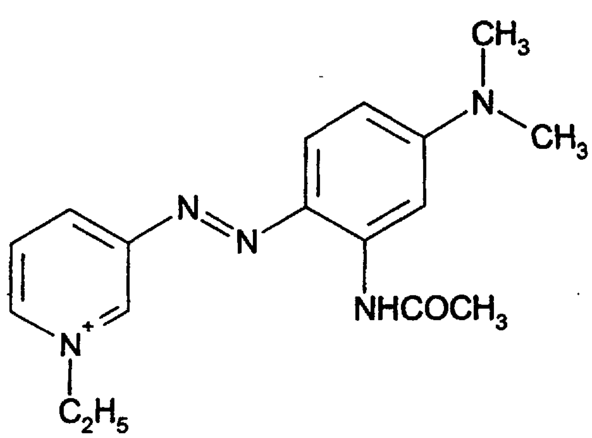

, $C_2H_5SO_4^-$

;

- le méthosutfate de diamino-2',4'-méthoxy-5'-benzène-azo-1' : 3-méthyl pyridinium de formule

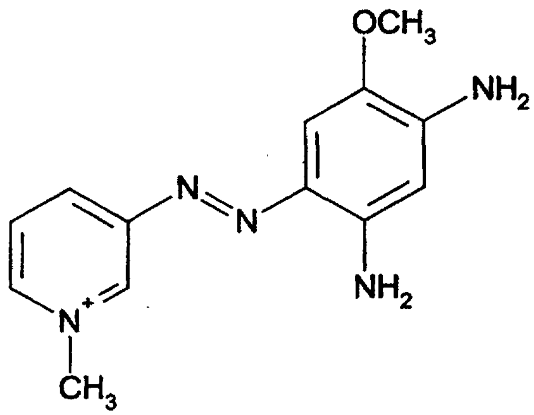

, $CH_3SO_4^-$

;

et

- le méthosulfate d'amino-2'-diméthylamino-4'-benzène-azo-1' : 3-méthyl-1 pyridinium de formule

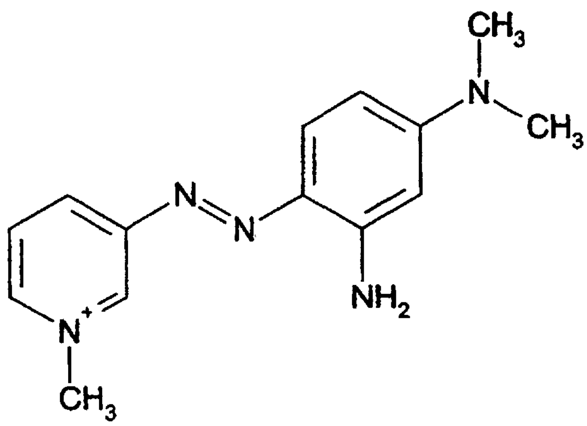

, $CH_3SO_4^-$

.

Le ou les dérivés de 3-amino pyridine de formule (I) utilisés selon l'invention, représentent de préférence de 0,001 à 10 % en poids environ du poids total de la composition tinctoriale et encore plus préférentiellement de 0,01 à 5 % en poids environ de ce poids.

La composition tinctoriale conforme à l'invention peut en outre contenir un ou plusieurs coupleurs et/ou une ou plusieurs bases d'oxydation benzéniques et/ou un ou des colorants directs différents des dérivés de 3-amino pyridine de formule (I), notamment pour modifier les nuances ou les enrichir en reflets.

Parmi les coupleurs pouvant être présents dans la composition tinctoriale conforme à l'invention, on peut notamment citer les méta-phénylènediamines, les méta-aminophénols, les métadiphénols, les coupleurs hétérocycliques, et leurs sels d'addition avec un acide.

Lorsqu'ils sont présents ces coupleurs additionnels représentent de préférence de 0,0001 à 10 % en poids environ du poids total de la composition tinctoriale et encore plus préférentiellement de 0,005 à 5 % en poids environ de ce poids.

Parmi les bases d'oxydation benzéniques pouvant être présentes à titre additionnel dans la composition tinctoriale conforme à l'invention, on peut notamment citer les paraphénylènediamines, les para-aminophénols, les orthoaminophénols et les bases doubles telles que les bis-phénylalkylènediamines.

Lorsqu'elles sont présentes, ces bases benzéniques représentent de préférence de 0,0005 à 12 % en poids environ du poids total de la composition tinctoriale conforme à l'invention, et encore plus préférentiellement de 0,005 à 6 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Le milieu approprié pour la teinture (ou support) de la composition tinctoriale conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en $C_1$-$C_4$, tels que l'éthanol et l'isopropanol.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 12 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux
ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (II) suivante :

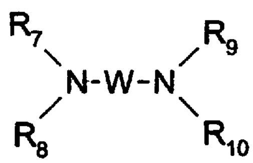

(II)

dans laquelle W est un reste propylène éventuellement substitué par un groupement hydroxyle ou un radical alkyle en $C_1$-$C_4$ ; $R_7$, $R_8$, $R_9$ et $R_{10}$, identiques ou différents, représentent un atome d'hydrogène, un radical alkyle en $C_1$-$C_4$ ou hydroxyalkyle en $C_1$-$C_4$.

La composition tinctoriale conforme à l'invention peut également renfermer divers adjuvants utilisés classiquement dans les compositions pour la teinture des cheveux.

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées.

La composition tinctoriale conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains.

L'invention a également pour objet un procédé de teinture des fibres kératiniques, et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, les peracides, les enzymes telles que les oxydo-réductases à 2 électrons, les peroxydases et les laccases. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

**EXEMPLES**

<u>EXEMPLES DE TEINTURE COMPARATIFS 1 A 4</u>

On a préparé les compositions tinctoriales suivantes (teneurs en grammes) :

| EXEMPLE | 1 | 2(*) | 3 | 4 (*) |
|---|---|---|---|---|
| Diméthylamino-4'-benzène-azo-1': 3-pyridine N-oxyde (composé de formule (I)) | 0,5 | 0,5 | - | - |
| Ethosulfate d'acétylamino-2'-diméthylamino-4'-benzène-azo-1' : 3-éthyl-1-pyridinium (composé de formule (I)) | - | - | 0,6 | 0,6 |
| Dichlorhydrate de 4,5-diamino 1-éthyl 3-méthyl pyrazole (base d'oxydation hétérocyclique) | 0,639 | - | 0,639 | - |
| Paraphénylènediamine (base d'oxydation benzénique) | - | 0,324 | - | 0,324 |
| Méta-aminophénol (coupleur) | 0,327 | 0,327 | 0,327 | 0,327 |
| Support de teinture commun | (**) | (**) | (**) | (**) |
| Eau déminéralisée qsp | 100 g | 100 g | 100 g | 100 g |

(*) : Exemple comparatif ne faisant pas partie de l'invention

(**) : <u>Support de teinture commun</u> :
- Alcool oléique polyglycérolé à 2 moles de glycérol 4,0 g
- Alcool oléique polyglycérolé à 4 moles de glycérol à 78 % de matières actives (M.A.) 5,69 g M.A.
- Acide oléique 3,0 g
- Amine oléique à 2 moles d'oxyde d'éthylène vendue sous la dénomination commerciale ETHOMEEN O12® par la société AKZO 7,0 g
- Laurylamino succinamate de diéthylaminopropyle, sel de sodium à 55 % de M.A. 3,0 g M.A.
- Alcool oléique 5,0 g
- Diéthanolamide d'acide oléique 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium à en solution aqueuse à 35 % de M.A. 0,455 g M.A.
- Acétate d'ammonium 0,8 g
- Antioxydant, séquestrant q.s.
- Parfum, conservateur q.s.
- Ammoniaque à 20 % de $NH_3$ 10,0 g

Au moment de l'emploi, on a mélangé chacune des compositions tinctoriales décrites ci-dessus avec une quantité pondérale équivalente de peroxyde d'hydrogène à 20 volumes (6% en poids) présentant un pH d'environ 3.

Chaque mélange résultant présentait un pH d'environ 10 ± 0,2 et a été appliqué pendant 30 minutes sur des mèches de cheveux gris à 90 % de blancs permanentés.

Les cheveux ont ensuite été rincés à l'eau, lavés avec un shampooing standard, rincés à nouveau puis séchés.
La couleur des mèches a été évaluée avant et après la teinture, dans le système Munsell, au moyen d'un spectrophotomètre CM 2002 MINOLTA ®.
Selon la notation Munsell, une couleur est définie par la formule :

$$HV/C$$

dans laquelle les trois paramètres désignent respectivement la "Hue" ou nuance (H), la "Value" ou intensité (V) et le "Chroma" ou saturation (C), la barre oblique étant simplement une convention et ne désignant pas un ratio.
La montée de la coloration $\Delta E$ peut être calculée en appliquant l'équation de Nickerson :

$$\Delta E = 0.4C_0dH + 6dV + 3dC$$

telle que décrite par exemple dans "Journal of the Optical Society of America", vol.34, N°. 9, Sept 1944, pages 550-570.
Dans cette équation, $\Delta E$ représente la différence de couleur entre deux mèches, (dans le cas présent la montée de la coloration), dH, dV, et dC représente la variation en valeur absolue des trois paramètres H, V, et C, $C_o$ représentant la saturation de la mèche par rapport à laquelle on veut évaluer la différence de couleur.
Plus la valeur de $\Delta E$ est importante, plus la différence de couleur entre les deux mèches est importante, et dans le cas présent, plus la montée (puissance) de la coloration est importante.
Les résultats figurent dans le tableau ci-après :

| Exemple | Couleur de la mèche avant teinture | Couleur de la mèche après teinture | Montée de la coloration | | | |
|---|---|---|---|---|---|---|
| | | | dH | dV | dC | ΔE |
| 1 | 3,3 Y 5,8 / 1,6 | 7,5 R 2,7 / 3,5 | 15,8 | 3,1 | 1,9 | **34,4** |
| 2 (*) | 3,3 Y 5,8 / 1,6 | 1,2 YR 2,4 / 2,1 | 12,1 | 3,4 | 0,5 | **29,6** |
| 3 | 3,3 Y 5,8 / 1,6 | 1,5 R 2,6 / 3,3 | 21,8 | 3,2 | 1,7 | **38,3** |
| 4 (*) | 3,3 Y 5,8 / 1,6 | 8,7 R 2,2 / 1,5 | 14,6 | 3,6 | 0,1 | **31,2** |

(*) exemple comparatif ne faisant pas partie de l'invention

On constate que les compositions tinctoriales des exemples 1 et 3 conformes à l'invention, c'est à dire contenant l'association d'un colorant direct de formule (I), d'une base d'oxydation hétérocyclique et d'un coupleur conduisent à des colorations plus puissantes que les compositions tinctoriales des exemples 2 et 4 ne faisant pas partie de l'invention dans la mesure où elles contiennent base d'oxydation benzénique à la place de la base d'oxydation hétérocyclique, et telles que décrites par exemple dans la demande de brevet FR-A-2 285 851.

## Revendications

1. Composition pour la teinture d'oxydation des fibres kératiniques et en particulier des fibres kératiniques humaines telles que les cheveux, **caractérisée par le fait qu'**elle comprend, dans un milieu approprié pour la teinture :

   - au moins une base d'oxydation hétérocyclique, et
   - à titre de colorant direct, au moins un dérivé de 3-amino pyridine choisi parmi les composés de formule (I) suivante:

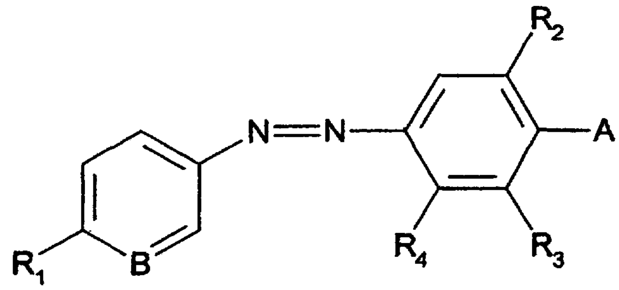

(I)

dans laquelle :

- B représente un groupement de formules (Ia) ou (Ib) suivantes :

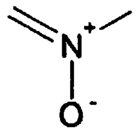

(Ia)

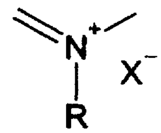

(Ib)

- R représente un radical alkyle en $C_1$-$C_4$ ;
- $R_1$ représente un atome d'hydrogène ou d'halogène tel que le chlore, le brome ou le fluor, un radical alkyle en $C_1$-$C_4$ ou alcoxy en $C_1$-$C_4$ ;
- $R_2$ représente un atome d'hydrogène, un radical alkyle en $C_1$-$C_4$ ou alcoxy en $C_1$-$C_4$ ;
- $R_4$ représente un atome d'hydrogène ou d'halogène tel que le chlore, le brome ou le fluor, un radical alkyle en $C_1$-$C_4$, nitro, amino ou acyl($C_1$-$C_4$)amino ;
- $R_3$ représente un atome d'hydrogène ou bien $R_4$ et $R_3$ forment un cycle insaturé à 6 chaînons portant un substituant hydroxyle chélaté avec un des atomes d'azote de la double liaison azoïque :

- A représente un reste -$NR_5R_6$ dans lequel $R_5$ représente un atome d'hydrogène, un radical alkyle en $C_1$-$C_4$, monohydroxyalkyle en $C_1$-$C_4$ ou polyhydroxyalkyle en $C_2$-$C_4$ ; $R_6$ représente un atome d'hydrogène, un radical alkyle en $C_1$-$C_4$, monohydroxyalkyle en $C_1$-$C_4$ ou polyhydroxyalkyle en $C_2$-$C_4$, un cycle phényle ou un radical -$CH_2$-$SO_3Na$;
- $X^-$ représente un anion monovalent ou divalent et est de préférence choisi parmi un atome d'halogène tel que le chlore, le brome, le fluor ou l'iode, un hydroxyde, un hydrogènesulfate, ou un alkyl($C_1$-$C_6$)sulfate tel que par exemple un méthylsulfate ou un éthylsulfate.

**2.** Composition selon la revendication 1, **caractérisée par le fait que** la ou les bases d'oxydation sont choisies parmi les dérivés pyridiniques, les dérivés pyrimidiniques, les dérivés pyrazoliques, et leurs sels d'addition avec un acide.

**3.** Composition selon la revendication 2, **caractérisée par le fait que** les dérivés pyridiniques sont choisis parmi la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

**4.** Composition selon la revendication 2, **caractérisée par le fait que** les dérivés pyrimidiniques sont choisis parmi la 2,4,5,6-tétra-aminopyrimidine, la 4-hydroxy 2,5,6-triaminopyrimidine, la pyrazolo-[1,5-a]-pyrimidine-3,7-diamine, la 2-méthyl pyrazolo-[1,5-a]-pyrimidine-3,7-diamine, la 2,5-diméthyl pyrazolo-[1,5-a]-pyrimidine-3,7-diamine, la pyrazolo-[1,5-a]-pyrimidine-3,5-diamine, la 2,7-diméthyl pyrazolo-[1,5-a]-pyrimidine-3,5-diamine, le 3-amino pyrazoto-[1,5-a]-pyrimidin-7-ol, le 3-amino 5-méthyl pyrazolo-[1,5-a]-pyrimidin-7-ol, le 3-amino pyrazolo-[1,5-a]-pyrimidin-5-ol, le 2-(3-amino pyrazolo-[1,5-a]-pyrimidin-7-ylamino)-éthanol, la 3-amino-7-β-hydroxyéthylamino-5-méthyl-pyrazolo-[1,5-a]-pyrimidine, le 2-(7-amino pyrazolo-[1,5-a]-pyrimidin-3-ylamino)-éthanol, le 2-[(3-amino-pyrazolo-[1,5-a]-pyrimidin-7-yl)-(2-hydroxyéthyl)-amino]-éthanol, le 2-[(7-amino-pyrazolo-[1,5-a]-pyrimidin-3-yl)-(2-hydroxyéthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N-7, N-7-tetraméthyl pyrazolo-[1,5-a]-pyrimidine-3,7-diamine, et leurs sels d'addition et leurs formes tautomères, lorsqu'il existe un équilibre tautomérique.

**5.** Composition selon la revendication 2, **caractérisée par le fait que** les dérivés pyrazoliques sont choisis parmi le 4,5-diamino pyrazole, le 4,5-diamino 1-méthyl pyrazole, le 1-benzyl 4,5-diamino pyrazole, le 3,4-diamino pyrazole, le 1-benzyl 4,5-diamino 3-méthyl pyrazole, le 4-amino 1,3-diméthyl 5-hydrazino pyrazole, le 4,5-diamino 3-méthyl 1-phényl pyrazole, le 4,5-diamino 3-méthyl 1-tert-butyl pyrazole, le 4,5-diamino 1-méthyl 3-tert-butyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, et leurs sels d'addition avec un acide.

**6.** Composition selon l'une quelconque des revendications précédentes, **caractérisée par le fait que** la ou les bases d'oxydation hétérocyclique représentent de 0,0005 à 12 % en poids du poids total de la composition tinctoriale.

**7.** Composition selon la revendication 6, **caractérisée par le fait que** la ou les bases d'oxydation hétérocyclique représentent de 0,005 à 6 % en poids du poids total de la composition tinctoriale.

**8.** Composition selon l'une quelconque des revendications précédentes, **caractérisée par le fait que** le ou les dérivés de 3-amino pyridine de formule (I) sont choisis parmi :

- le méthosulfate de diméthylamino-4' benzène azo-1' : 3-méthyl-1-pyridinium de formule :

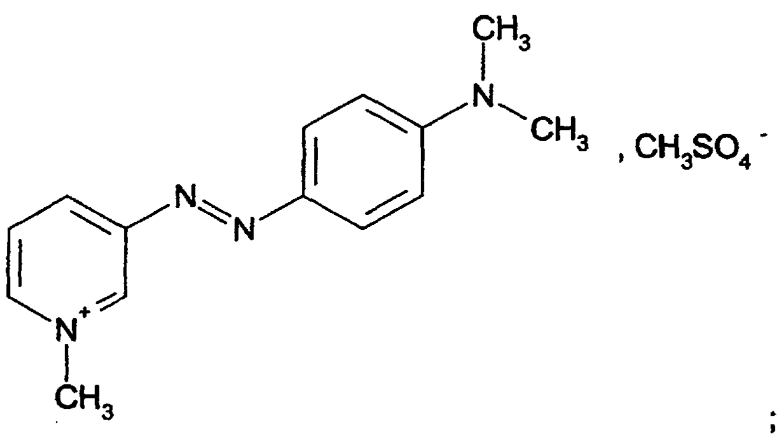

;

- le méthosulfate de bis-(β-hydroxyéthyl)amino-4'-benzène-azo-1' : 3-méthyl-1'-pyridinium de formule :

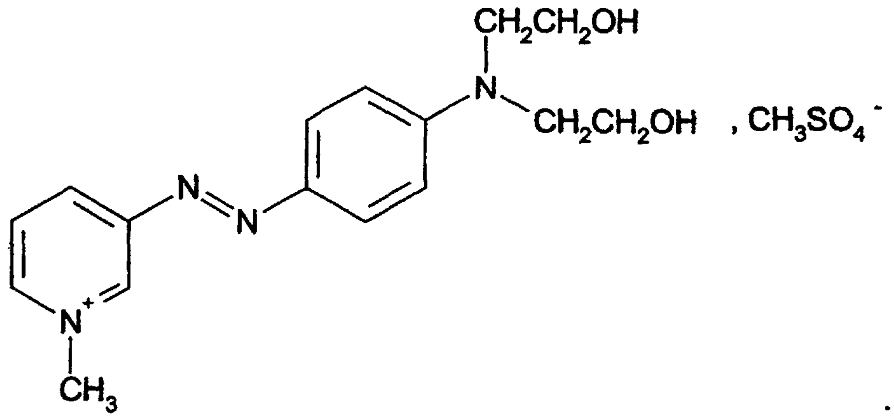

;

- le méthosulfate d'amino-4'-hydroxy-8'-naphtalène-azo-1' : 3-méthyl-1-pyridinium de formule

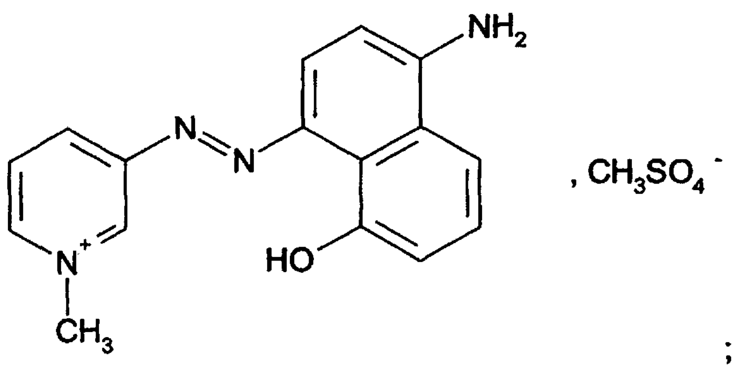

;

- le méthosulfate de diméthylamino-4'-nitro-2'-benzène-azo-1' : 3-méthyl-1-pyridinium de formule

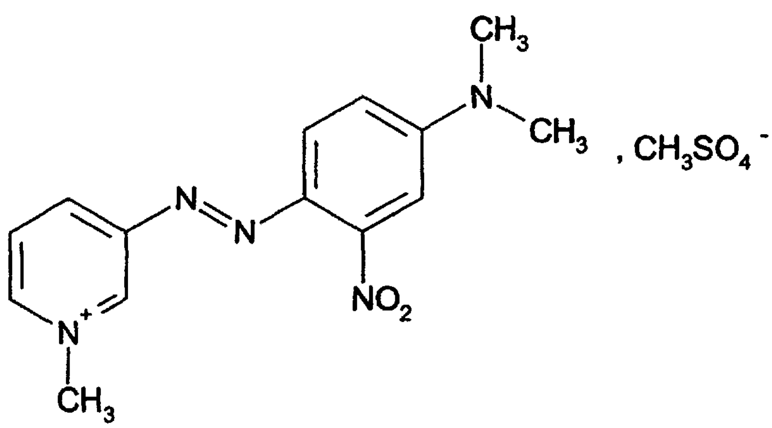

;

- le méthosulfate de diméthylamino-4'-benzène-azo-1' : 3-diméthyl-1,6-pyridinium de formule :

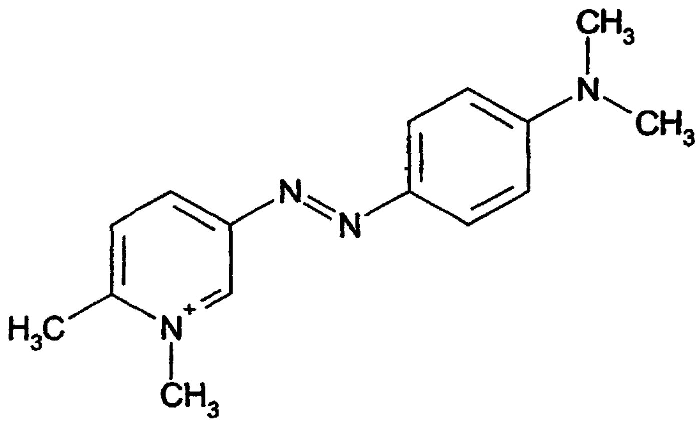

, $CH_3SO_4^-$

;

- l'amino-4'-benzène-azo-1' : 3-pyridine N-oxyde de formule

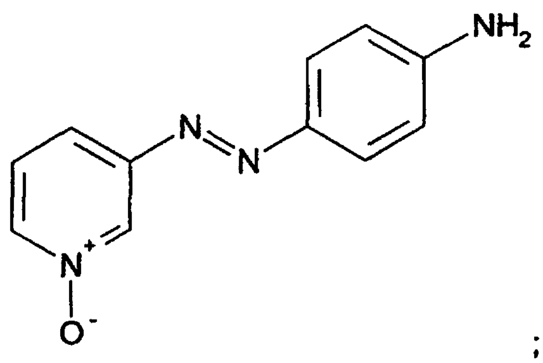

;

- la diméthylamino-4'-benzène-azo-1' : 3-pyridine N-oxyde de formule

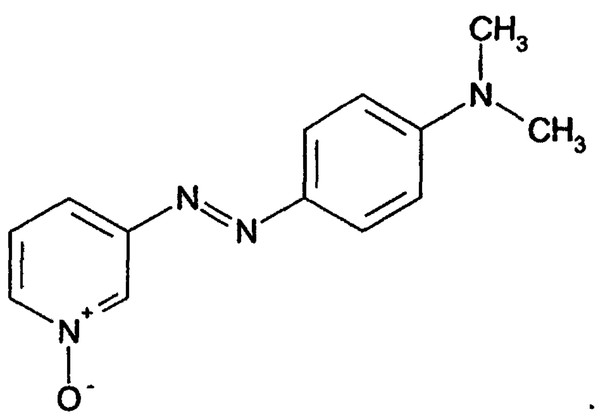

;

- la N,N-bis-(β-hydroxyéthyl)amino-4'-benzène-azo-1' : 3-pyridine N-oxyde de formule:

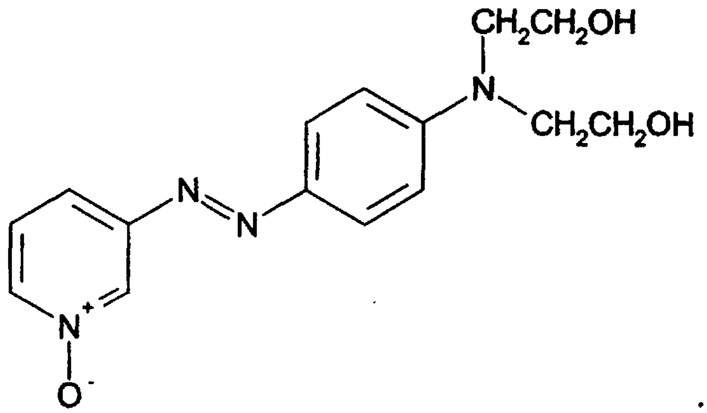

;

- l'éthosulfate de diméthylamino-4'-méthyl-2'-benzène-azo-1' : 3-éthyl-1-pyridinium de formule :

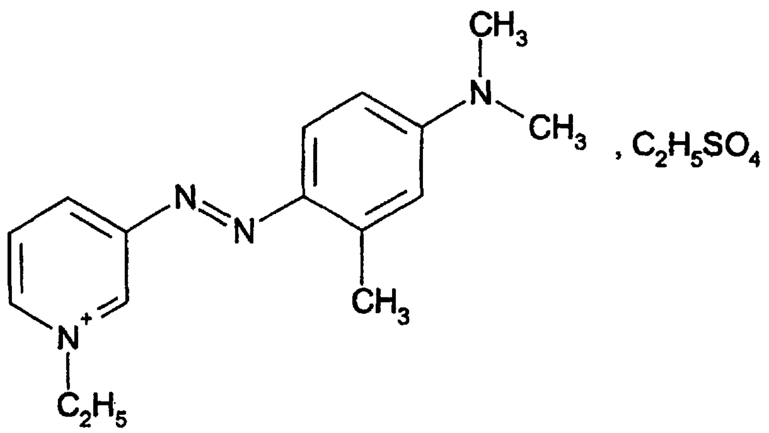

;

- le bromure de diméthylamino-4'-méthyl-2'-benzène-azo-1': 3-butyl-1-pyridinium de formule:

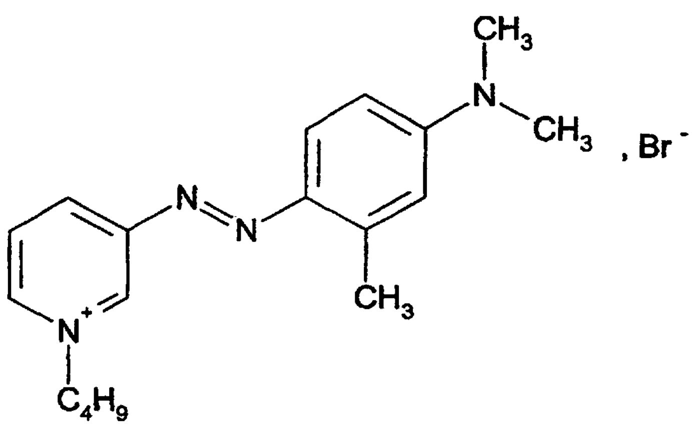

;

- le méthosulfate de diméthylamino-4'-chloro-2'-benzène-azo-1' : 3-méthyl-1-pyridinium de formule :

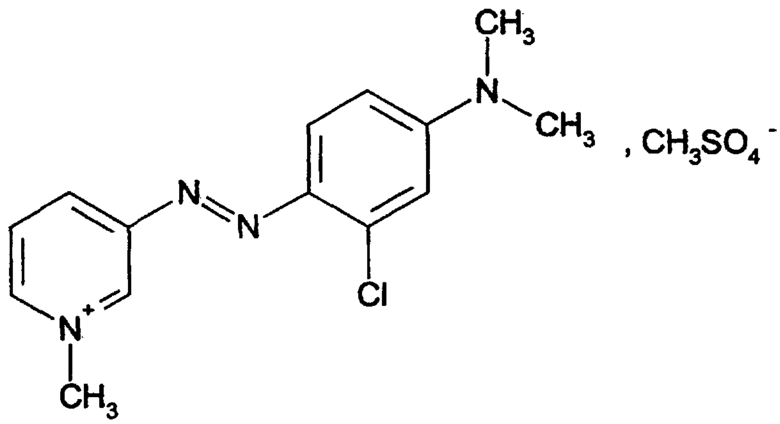

;

- le méthosulfate de diamino-2',4'-méthyl-5'-benzène-azo-1' : 3-méthyl-1 pyridinium de formule :

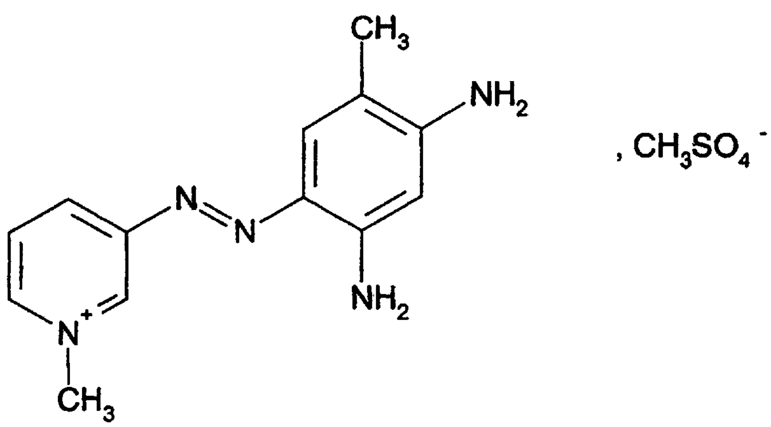

;

- le méthosulfate de phénylamino-4'-benzène-azo-1' : 3-méthyl-1-pyridinum de formule

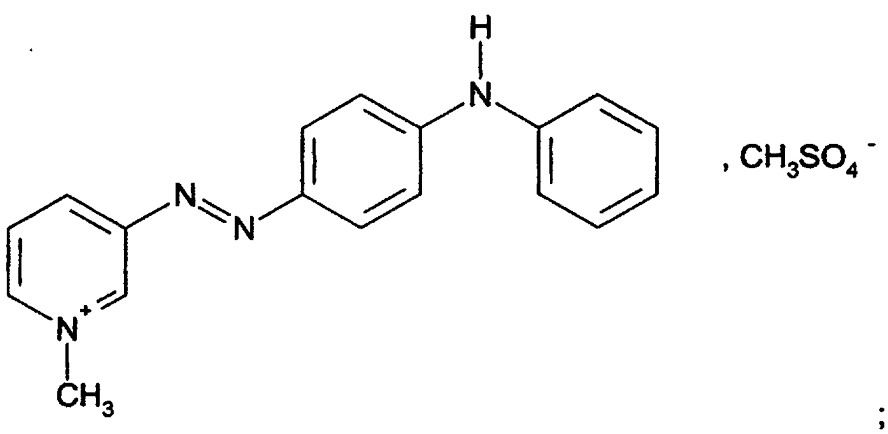

;

- l'éthosulfate d'acétylamino-2'-diméthylamino-4'-benzène-azo-1' : 3-éthyl-1-pyridinium de formule

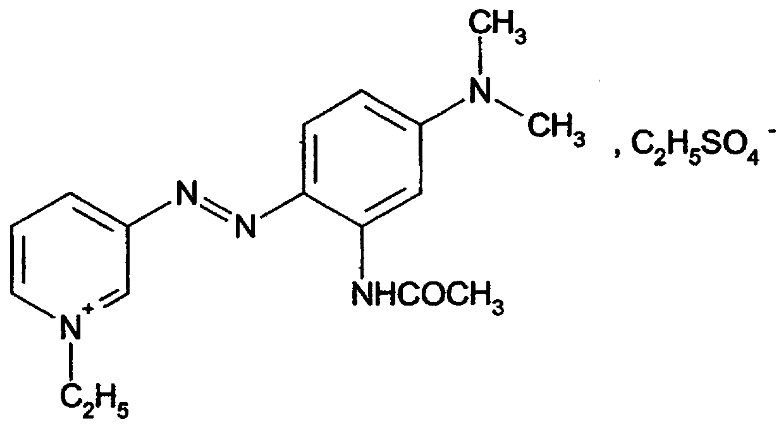

;

- le méthosulfate de diamino-2',4'-méthoxy-5'-benzène-azo-1' : 3-méthyl pyridinium de formule

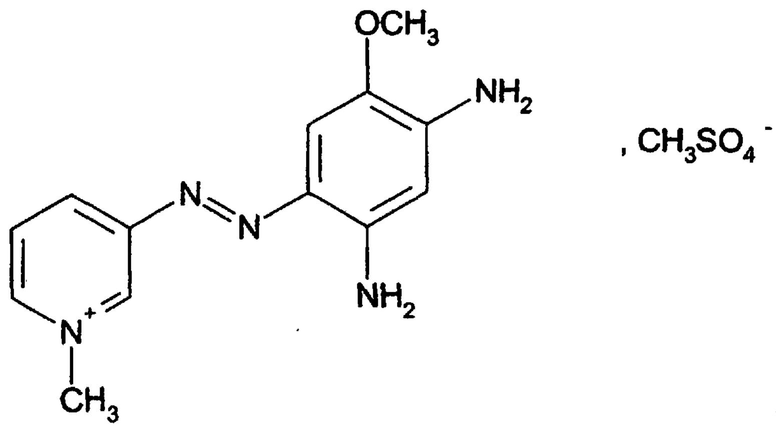

;

et

- le méthosulfate d'amino-2'-diméthylamino-4'-benzène-azo-1' : 3-méthyl-1 pyridinium de formule :

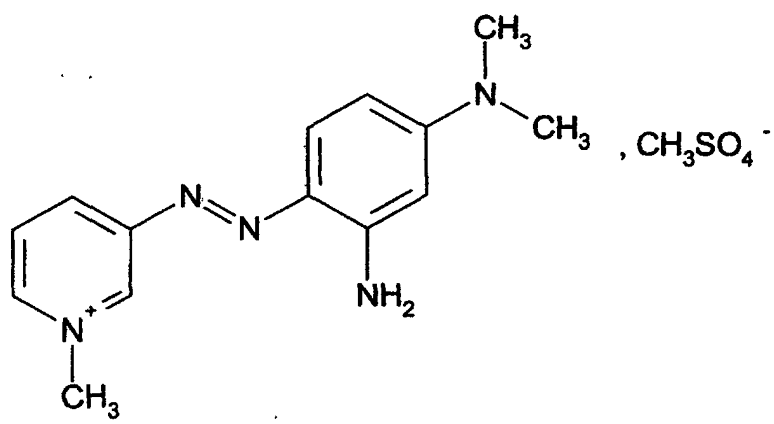

.

**9.** Composition selon l'une quelconque des revendications précédentes, **caractérisée par le fait que** le ou les dérivés de 3-amino pyridine de formule (I) représentent de 0,001 à 10 % en poids du poids total de la composition tinctoriale.

**10.** Composition selon la revendication 9, **caractérisée par le fait que** le ou les dérivés de 3-amino pyridine de formule

(I) représentent de 0,01 à 5 % en poids du poids total de la composition tinctoriale.

**11.** Composition selon l'une quelconque des revendications précédentes, **caractérisée par le fait qu'**elle renferme un ou plusieurs coupleurs et/ou une ou plusieurs bases d'oxydation benzéniques et/ou un ou des colorants directs différents des dérivés de 3-amino pyridine de formule (I) tels que définis à la revendication 1.

**12.** Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétates.

**13.** Composition selon l'une quelconque des revendications précédentes, **caractérisée par le fait que** le milieu approprié pour la teinture est constitué par de l'eau ou par un mélange d'eau et d'au moins un solvant organique.

**14.** Composition selon l'une quelconque des revendications précédentes, **caractérisée par le fait qu'**elle présente un pH compris 3 et 12.

**15.** Procédé de teinture des fibres kératiniques et en particulier des fibres kératiniques humaines telles que les cheveux, **caractérisé par le fait qu'**on applique sur lesdites fibres au moins une composition tinctoriale telle que définie à l'une quelconque des revendications 1 à 14, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

**16.** Procédé selon la revendication 15, **caractérisé par le fait que** l'agent oxydant présent dans la composition oxydante est choisi parmi le peroxyde d'hydrogène, le peroxyde d'urée, les bromates de métaux alcalins, les persels tels que les perborates, les percarbonates et persulfates, les peracides, et les enzymes.

**17.** Dispositif à plusieurs compartiments, ou "kit" de teinture à plusieurs compartiments, dont un premier compartiment renferme une composition tinctoriale telle que définie à l'une quelconque des revendications 1 à 14 et un second compartiment renferme une composition oxydante.

**Patentansprüche**

**1.** Zusammensetzung zum oxidativen Färben von Keratinfasern und insbesondere menschlichen Keratinfasern, wie dem Haar, **dadurch gekennzeichnet, dass** sie in einem zum Färben geeigneten Medium enthält:

- mindestens eine heterocyclische Oxidationsbase, und
- als Direktfarbstoff mindestens ein 3-Amino-pyridinderivat, das unter den Verbindungen der folgenden Formel (I) ausgewählt ist:

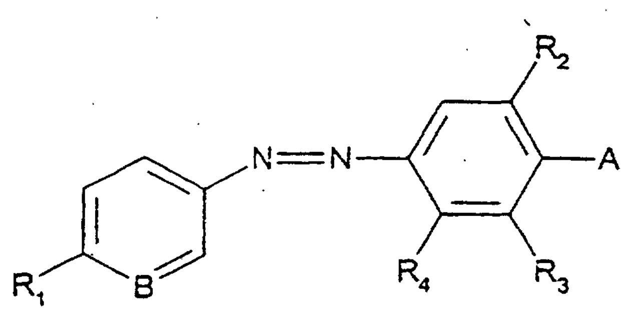

(I)

worin bedeuten:

- B eine Gruppe der folgenden Formeln (Ia) oder (Ib):

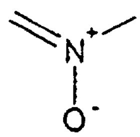 (Ia)

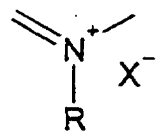 (Ib)

- R eine $C_{1-4}$-Alkylgruppe;
- $R_1$ ein Wasserstoffatom, ein Halogenatom, wie Chlor, Brom oder Fluor, eine $C_{1-4}$-Alkylgruppe oder eine $C_{1-4}$-Alkoxygruppe;
- $R_2$ ein Wasserstoffatom, eine $C_{1-4}$-Alkylgruppe oder eine $C_{1-4}$-Alkoxygruppe;
- $R_4$ ein Wasserstoffatom, ein Halogenatom, wie Chlor, Brom oder Fluor, eine $C_{1-4}$-Alkylgruppe, Nitro, Amino oder eine $C_{1-4}$-Acylaminogruppe;
- $R_3$ ein Wasserstoffatom oder die Gruppen $R_4$ und $R_3$ bilden einen ungesättigen 6-gliedrigen Ring, der einen Hydroxysubstituenten trägt, der ein Chelat mit den beiden Stickstoffatomen der Azo-Doppelbindung bildet;
- A eine Gruppe $-NR_5R_6$, wobei $R_5$ Wasserstoff, $C_{1-4}$-Alkyl, $C_{1-4}$-Monohydroxyalkyl oder $C_{2-4}$-Polyhydroxyalkyl und $R_6$ Wasserstoff, $C_{1-4}$-Alkyl, $C_{1-6}$-Monohydroxyalkyl, $C_{2-6}$-Polyhydroxyalkyl, Phenyl oder $-CH_2-SO_3Na$ bedeutet;
- die Gruppe $X^-$ bedeutet ein einwertiges oder zweiwertiges Anion,

wobei sie vorzugsweise unter den Halogenatomen, wie Chlor, Brom, Fluor oder Iod, Hydroxid, Hydrogensulfat oder Alkyl($C_{1-6}$)-sulfat, beispielsweise Methyl- oder Ethylsulfat, ausgewählt ist.

**2.** Zusammensetzung nach Anspruch 1, **dadurch gekennzeichnet, dass** die Oxidationsbase(n) unter den Pyridinderivaten, Pyrimidinderivaten, Pyrazolderivaten und deren Additionssalze mit einer Säure ausgewählt sind.

**3.** Zusammensetzung nach Anspruch 2, **dadurch gekennzeichnet, dass** die Pyridinderivate unter 2,5-Diamino-pyridin, 2-(4-Methoxyphenyl)-amino-3-amino-pyridin, 2,3-Diamino-6-methoxy-pyridin, 2-(β-Methoxyethyl)amino-3-amino-6-methoxy-pyridin, 3,4-Diamino-pyridin und deren Additionssalze mit einer Säure ausgewählt sind.

**4.** Zusammensetzung nach Anspruch 2, **dadurch gekennzeichnet, dass** die Pyrimidinderivate unter 2,4,5,6-Tetraaminopyrimidin, 4-Hydroxy-2,5,6-triaminopyrimidin, Pyrazolo-[1,5-a]-pyrimidin-3,7-diamin, 2-Methyl-pyrazolo-[1,5-a]-pyrimidin-3,7-diamin, 2,5-Dimethyl-pyrazolo-[1,5-a]-pyrimidin-3,7-diamin, Pyrazob-[1,5-a]-pyrimidin-3,5-diamin, 2,7-Dimethyl-pyrazolo-[1,5-a]-pyrimidin-3,5-diamin, 3-Amino-pyrazolo-[1,5-a]-pyrimidin-7-ol, 3-Amino-5-methylpyrazolo-[1,5-a]-pyrimidin-7-ol, 3-Amino-pyrazolo-[1,5-a]-pyrimidin-5-ol, 2-(3-Amino-pyrazolo-[1,5-a]-pyrimidin-7-ylamino)-ethanöl, 3-Amino-7-β-hydroxyethylamino-5-methyl-pyrazolo-[1,5-a]-pyrimidin, 2-(7-Amino-pyrazolo-[1,5-a]-pyrimidin-3-ylamino)-ethanol, 2-[(3-Amino-pyrazolo-[1,5-a]-pyrimidin-7-yl)-(2-hydroxyethyl)amino]-ethanol, 2-[(7-Amino-pyrazolo-[1,5-a]-pyrimidin-3-yl)-(2-hydroxyethyl)amino]-ethanol, 5,6-Dimethyl-pyrazolo-[1,5-a]-pyrimidin-3,7-diamin, 2,6-Dimethyl-pyrazolo-[1,5-a]-pyrimidin-3,7-diamin und 2,5,N7,N7-Tetramethyl-pyrazolo-[1,5-a]-pyrimidin-3,7-diainin und deren Additionssalzen mit einer Säure ausgewählt sind.

**5.** Zusammensetzung nach Anspruch 2, **dadurch gekennzeichnet, dass** die Pyrazolderivate unter 4,5-Diamino-pyrazol, 4,5-Diamino-1-methyl-pyrazol, 1-Benzyl-4,5-diamino-pyrazol, 3,4-Diamino-pyrazol, 1-Benzyl-4,5-diamino-3-methyl-pyrazol, 4-Amino-1,3-dimethyl-5-hydrazino-pyrazol, 4,5-Diamino-3-methyl-1-phenyl-pyrazol, 4,5-Diamino-3-methyl-1-*t*-butyl-pyrazol, 4,5-Diamino-1-methyl-3-*t*butyl-pyrazol, 4,5-Diamino-1-ethyl-3-methyl-pyrazol, 4,5-Diamino-1-ethyl-3-(4'-methoxyphenyl)-pyrazol, 4,5-Diamino-1-ethyl-3-hydroxymethyl-pyrazol, 4,5-Diamino-3-hydroxymethyl-1-methylpyrazol, 4,5-Diamino-3-hydroxymethyl-1-isopropyl-pyrazol, 4,5-Diamino-3-methyl-1-isopropyl-pyrazol und deren Additionssalze mit einer Säure ausgewählt sind.

**6.** Zusammensetzung nach einem der vorhergehenden Ansprüche, **dadurch gekennzeichnet, dass** die heterocyclische(n) Oxidationsbase(n) 0,0005 bis-12 Gew.-% des Gesamtgewichts der Farbmittelzusammensetzung ausmachen.

**7.** Zusammensetzung nach Anspruch 6, **dadurch gekennzeichnet, dass** die heterocyclische(n) Oxidationsbase(n) 0,005 bis 6 Gew.-% des Gesamtgewichts der Farbmittelzusammensetzung ausmachen.

**8.** Zusammensetzung nach einem der vorhergehenden Ansprüche, **dadurch gekennzeichnet, dass** das (die) 3-Amino-pyridinderivat(e) der Formel (I) unter den folgenden Verbindungen ausgewählt ist (sind):

- 3-[4'-Dimethylamino-1'-benzolazo]-1-methyl-pyridiniummethosulfat der folgenden Formel:

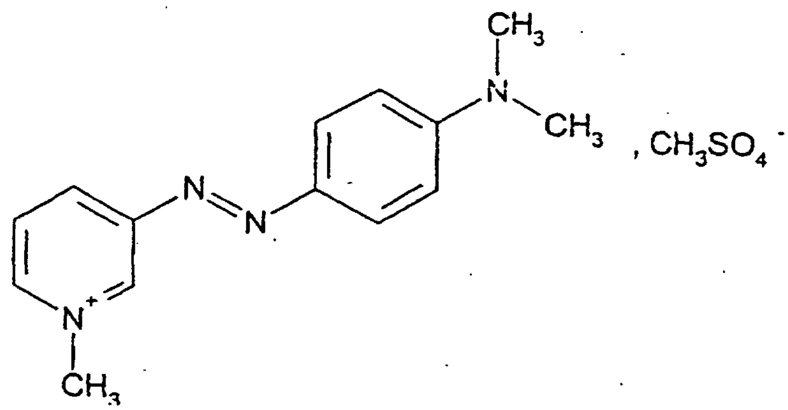

- 3-[4'-Bis(β-hydroxyethyl)amino-1'-benzolazo]-1-methyl-pyridiniummethosulfat der folgenden Formel:

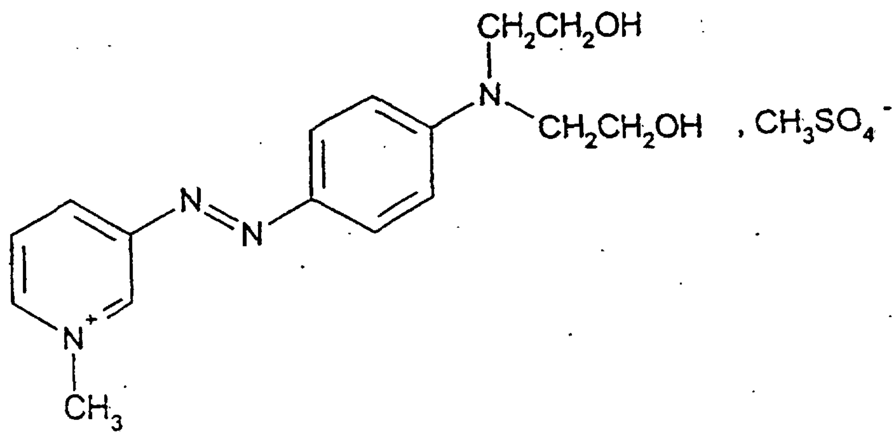

- 3-[4'-Amino-8'-hydroxy-1'-naphthalinazo]-1-methyl-pyridiniummethosulfat der folgenden Formel:

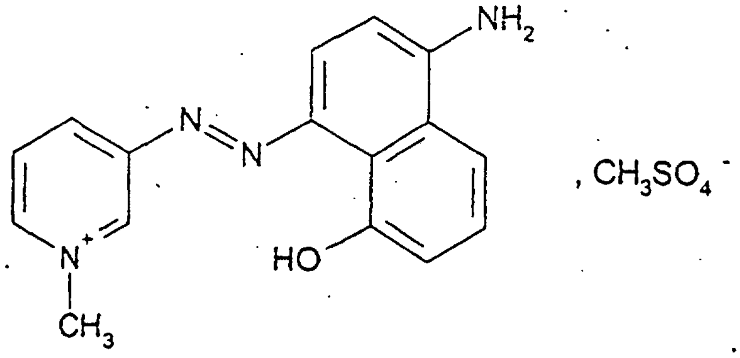

- 3-[4'-Dimethylamino-2'-nitro-1'-benzolazo]-1-methyl-pyridiniummethosulfat der folgenden Formel:

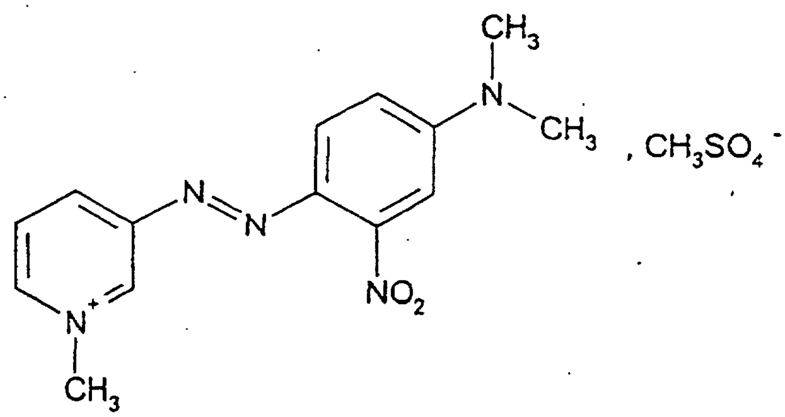

- 3-[4'-Dimethylamino-1'-benzolazo]-1,6-dimethyl-pyridiniummethosulfat der folgenden Formel:

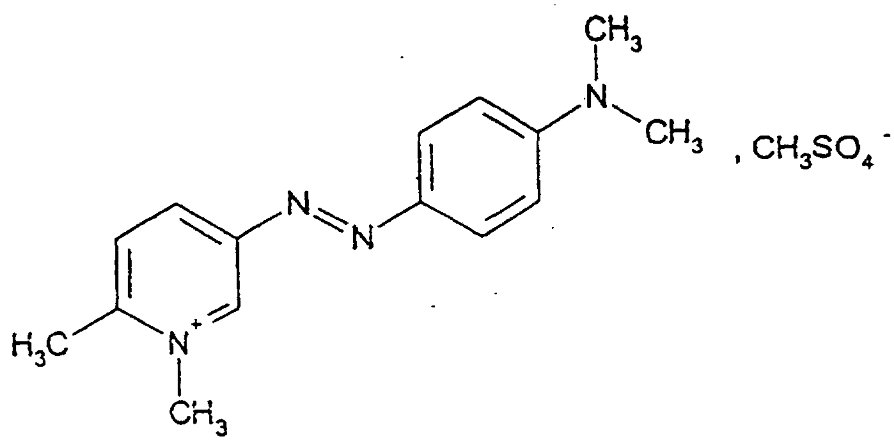

- 3-[4'-Amino-1'-benzolazo]-pyridin-N-oxid der folgenden Formel:

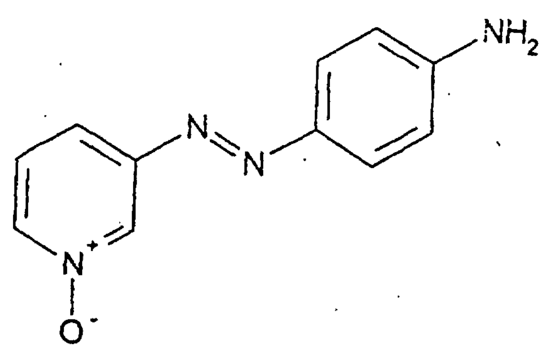

- 3-[4'-Dimethylamino-1'-benzolazo]-pyridin-N-oxid der folgenden Formel:

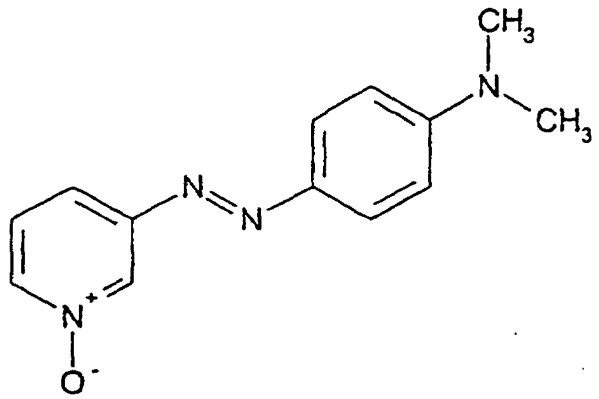

- 3-[4'-N,N-Bis(β-hydroxyethyl)amino-1'-benzolazo]-pyridin-N-oxid der folgenden Formel:

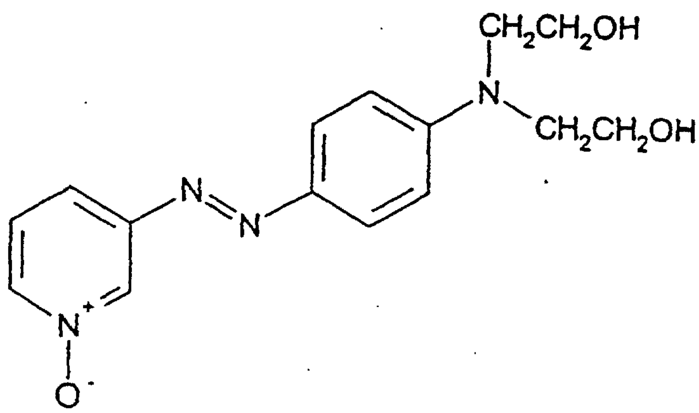

- 3-[4'-Dimethylamino-2'-methyl-1'-benzolazo]-1-ethyl-pyridinium-ethosulfat der folgenden Formel:

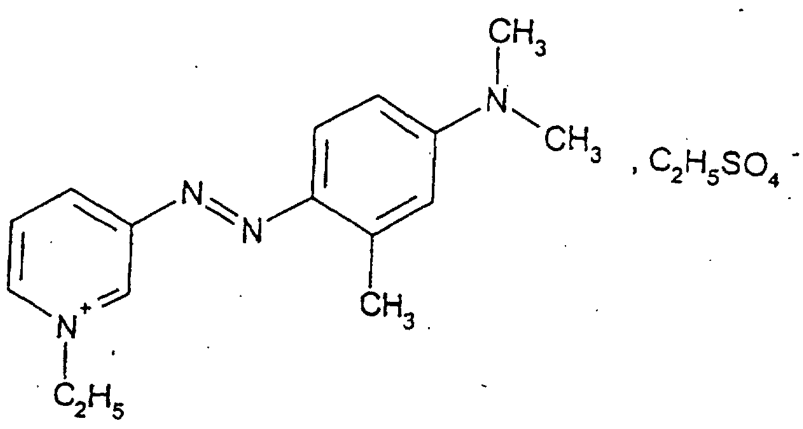

- 3-[4'-Dimethylamino-2'-methyl-1'-benzolazo]-1-butyl-pyridiniumbromid der folgenden Formel:

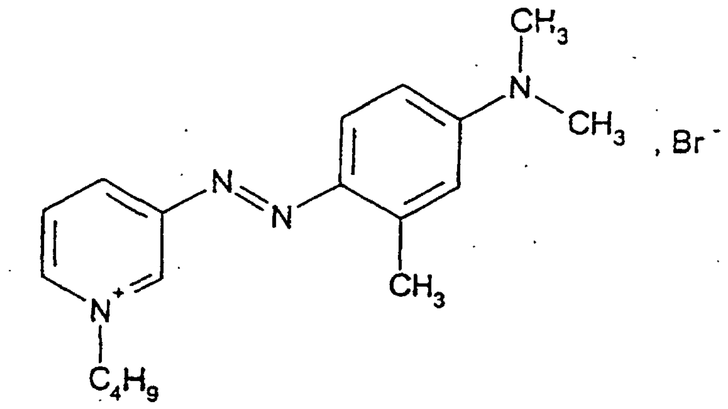

- 3-[4'-Dimethylamino-2'-Chlor-1'-benzolazo]-1-methyl-pyridiniummethosulfat der folgenden Formel:

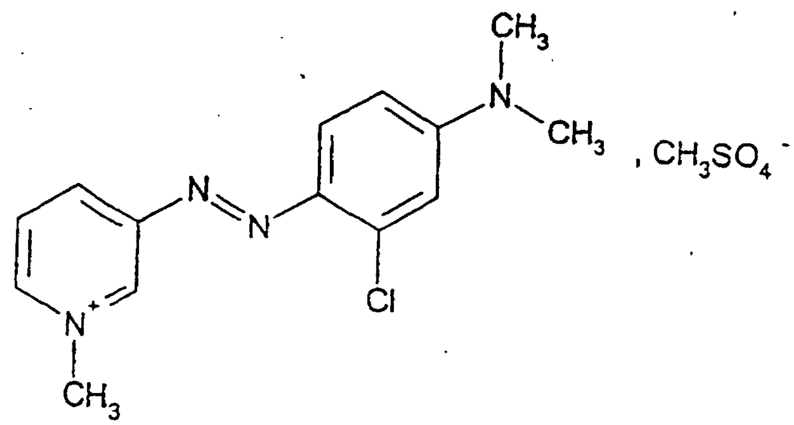

- 3-[2',4'-Diamino-5'-methyl-1'-benzolazo]-1-methyl-pyridiniummethosulfat der folgenden Formel:

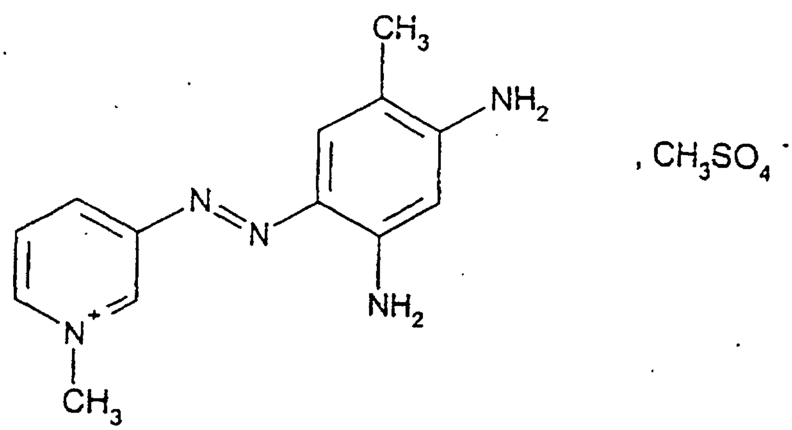

- 3-[4'-Phenylamino-1'-benzolazo]-1-methyl-pyridinium-methosulfat der folgenden Formel:

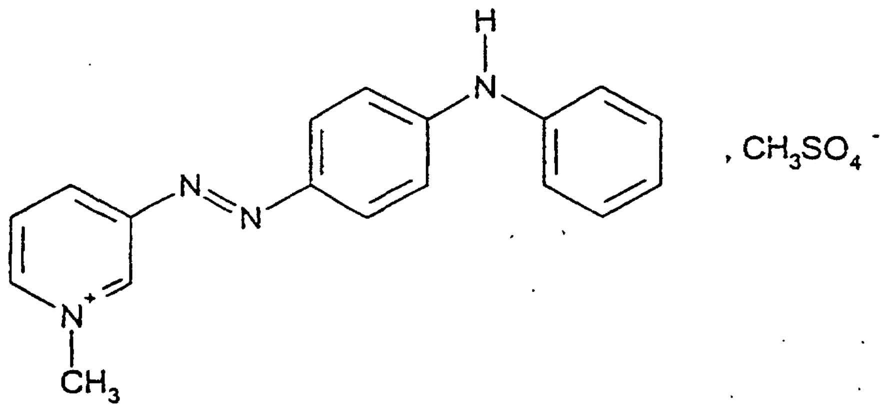

- 3-[2'-Acetylamino-4'-dimethylamino-1'-benzolazo]-1-ethylpyridinium-ethosulfat der folgenden Formel:

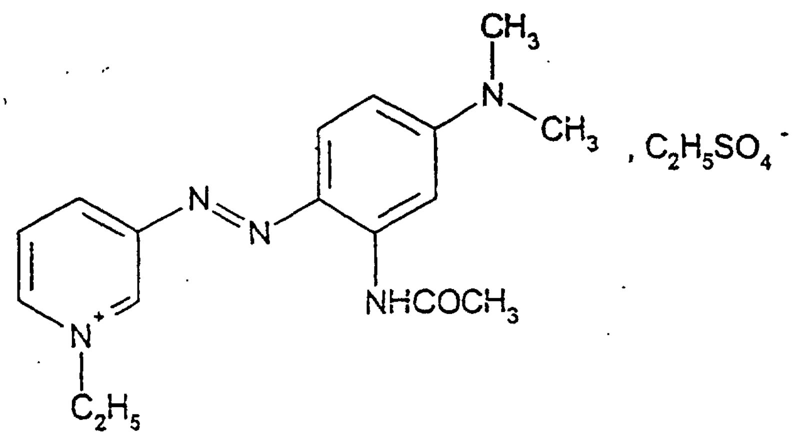

- 3-[2',4'-Diamino-5'-methoxy-1'-benzolazo]-1-methyl-pyridiniummethosulfat der folgenden Formel:

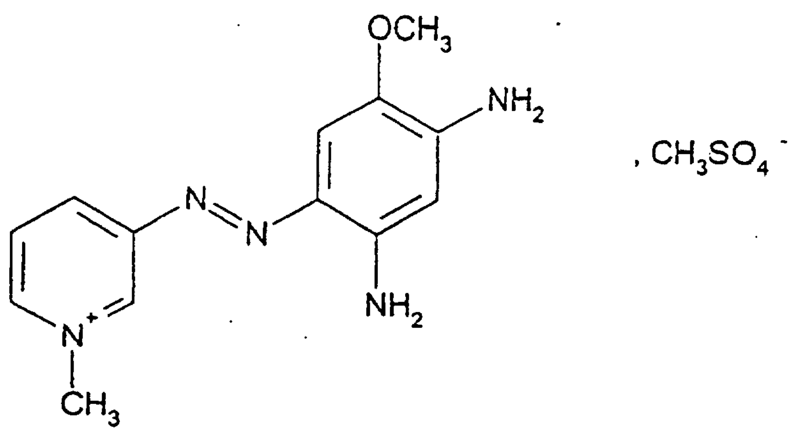

- 3-[2'-Amino-4'-Dimethylamino-1'-benzolazo]-1-methyl-pyridiniummethosulfat der folgenden Formel:

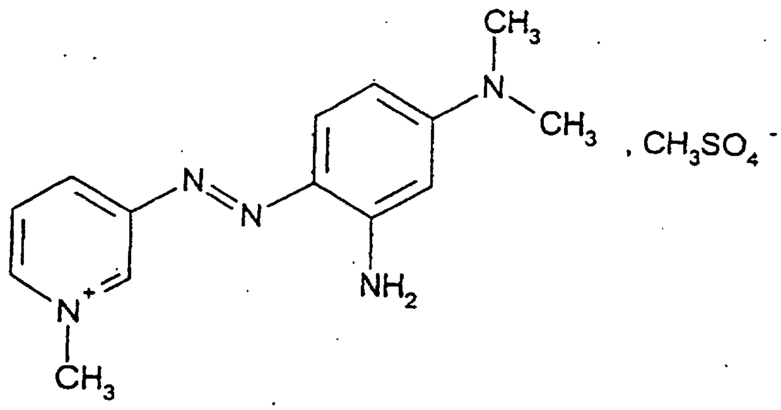

**9.** Zusammensetzung nach einem der vorhergehenden Ansprüche, **dadurch gekennzeichnet, dass** das (die) 3-Amino-pyridinderivat(e) der Formel (I) 0,001 bis 10 Gew.-% des Gesamtgewichts der Farbmittelzusammensetzung ausmachen.

**10.** Zusammensetzung nach einem der vorhergehenden Ansprüche, **dadurch gekennzeichnet, dass** das (die) 3-Amino-pyridinderivat(e) der Formel (I) 0,01 bis 5 Gew.-% des Gesamtgewichts der Farbmittelzusammensetzung ausmachen.

**11.** Zusammensetzung nach einem der vorhergehenden Ansprüche, **dadurch gekennzeichnet, dass** sie einen oder mehrere Kuppler und/oder eine oder mehrere Oxidationsbasen vom Benzoltyp und/oder einen oder mehrere Direktfarbstoffe, die von den 3-Aminopyridinderivaten der Formel (1) nach Anspruch 1 verschieden sind, enthält.

**12.** Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten ausgewählt sind.

**13.** Zusammensetzung nach einem der vorhergehenden Ansprüche, **dadurch gekennzeichnet, dass** das zum Färben geeignete Medium aus Wasser oder einem Gemisch von Wasser und mindestens einem organischen Lösungsmittel besteht.

**14.** Zusammensetzung nach einem der vorhergehenden Ansprüche, **dadurch gekennzeichnet, dass** sie einen pH-Wert im Bereich von 3 bis 12 aufweist.

**15.** Verfahren zum Färben von Keratinfasern und insbesondere menschlichen Keratinfasern, wie dem Haar, **dadurch gekennzeichnet, dass** auf die Fasern mindestens eine Farbmittelzusammensetzung nach einem der Ansprüche 1 bis 14 aufgebracht wird, wobei die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung vorliegt, die gleichzeitig oder getrennt davon anschließend aufgetragen wird.

**16.** Verfahren nach Anspruch 15, **dadurch gekennzeichnet, dass** das Oxidationsmittel unter Wasserstoffperoxid, Harnstoffperoxid, Alkalimetallbromaten, Salzen von Persäuren, wie Perboraten, Percarbonaten und Persulfaten, Persäuren und Enzymen ausgewählt ist.

**17.** Vorrichtung mit mehreren Abteilungen oder "Kit" zum Färben mit mehreren Abteilungen, wobei eine erste Abteilung eine Farbmittelzusammensetzung nach einem der Ansprüche 1 bis 14 und eine zweite Abteilung eine oxidierende Zusammensetzung enthält.

## Claims

1. Composition for the oxidation dyeing of keratin fibres, and in particular of human keratin fibres such as the hair, **characterized in that** it comprises, in a medium which is suitable for dyeing:

   - at least one heterocyclic oxidation base and
   - as direct dye, at least one 3-aminopyridine derivative chosen from the compounds of formula (I) below:

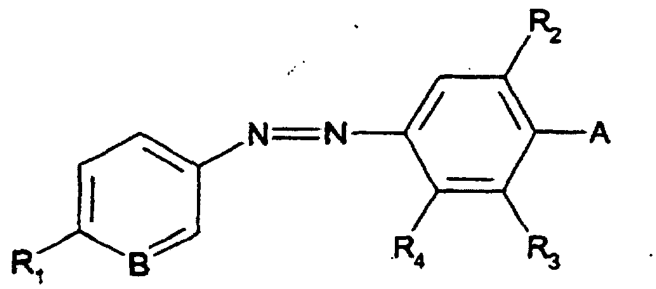
(I)

   in which:

   - B represents a group of formula (Ia) or (Ib) below:

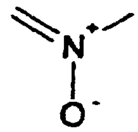
(Ia)

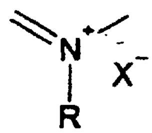
(Ib)

   - R represents a $C_1$-$C_4$ alkyl radical;
   - $R_1$ represents a hydrogen or halogen atom such as chlorine, bromine or fluorine, or a $C_1$-$C_4$ alkyl or $C_1$-$C_4$ alkoxy radical;
   - $R_2$ represents a hydrogen atom or a $C_1$-$C_4$ alkyl or $C_1$-$C_4$ alkoxy radical;
   - $R_4$ represents a hydrogen or halogen atom such as chlorine, bromine or fluorine, or a $C_1$-$C_4$ alkyl, nitro, amino or ($C_1$-$C_4$)acylamino radical;
   - $R_3$ represents a hydrogen atom or else $R_4$ and $R_3$ together form a 6-membered unsaturated ring bearing a hydroxyl substituent chelated with one of the nitrogen atoms of the azo double bond;
   - A represents a residue $-NR_5R_6$ in which $R_5$ represents a hydrogen atom or a $C_1$-$C_4$ alkyl, $C_1$-$C_4$ monohydroxyalkyl or $C_2$-$C_4$ polyhydroxyalkyl radical; $R_6$ represents a hydrogen atom, a $C_1$-$C_4$ alkyl, $C_1$-$C_4$ monohydroxyalkyl or $C_2$-$C_4$ polyhydroxyalkyl radical, a phenyl ring or a $-CH_2-SO_3Na$ radical;
   - $X^-$ represents a monovalent or divalent anion and is preferably chosen from a halogen atom such as chlorine, bromine, fluorine or iodine, a hydroxide, a hydrogen sulphate or a ($C_1$-$C_6$)alkyl sulphate such as, for example, a methyl sulphate or an ethyl sulphate.

2. Composition according to Claim 1, **characterized in that** the oxidation base(s) is (are) chosen from pyridine derivatives, pyrimidine derivatives and pyrazole derivatives, and the addition salts thereof with an acid.

3. Composition according to Claim 2, **characterized in that** the pyridine derivatives are chosen from 2,5-diaminopyridine, 2-(4-methoxyphenyl)amino-3-aminopyridine, 2,3-diamino-6-methoxypyridine, 2-(β-methoxyethyl)amino-3-amino-6-methoxypyridine and 3,4-diaminopyridine, and the addition salts thereof with an acid.

4. Composition according to Claim 2, **characterized in that** the pyrimidine derivatives are chosen from 2,4,5,6-tetraaminopyrimidine, 4-hydroxy-2,5,6-tri-aminopyrimidine, pyrazolo[1,5-a]pyrimidine-3,7-di-amine, 2-methylpyrazolo[1,5-a]pyrimidine-3,7-di-amine, 2,5-dimethylpyrazolo[1,5-a]pyrimidine-3,7-diamine, pyrazolo [1,5-a]pyrimidine-3,5-diamine, 2,7-dimethylpyrazolo[1,5-a]pyrimidine-3,5-diamine, 3-aminopyrazolo[1,5-a]pyrimidin-7-ol, 3-amino-5-methylpyrazolo[1,5-a]pyrimidin-7-ol, 3-aminopyrazolo[1,5-a]pyrimidin-5-ol, 2-(3-amino-pyrazolo[1,5-a]pyrimidin-7-ylamino)ethanol, 3-amino-7-β-hydroxyethylamino-5-methylpyrazolo[1,5-a]pyrimidine, 2-(7-aminopyrazolo[1,5-a]pyrimidin-3-ylamino)ethanol, 2-[(3-aminopyrazolo[1,5-a]pyrimidin-7-yl)-(2-hydroxyethyl)amino]ethanol, 2-[(7-aminopyrazolo[1,5-a]pyrimidin-3-yl)-(2-hydroxyethyl)amino]ethanol, 5,6-dimethylpyrazolo-[1,5-a]pyrimidine-3,7-diamine, 2,6-dimethyl-pyrazolo[1,5-a]pyrimidine-3,7-diamine and 2,5, N7,N7-tetramethylpyrazolo[1,5-a]pyrimidine-3,7-di-amine, and the addition salts thereof and the tautomeric forms thereof, when a tautomeric equilibrium exists.

5. Composition according to Claim 2, **characterized in that** the pyrazole derivatives are chosen from 4,5-diaminopyrazole, 4,5-diamino-1-methylpyrazole, 1-benzyl-4,5-diaminopyrazole, 3,4-diaminopyrazole, 1-benzyl-4,5-diamino-3-methylpyrazole, 4-amino-1,3-dimethyl-5-hydrazinopyrazole, 4,5-diamino-3-methyl-1-phenylpyrazole, 4,5-diamino-3-methyl-1-tert-butylpyrazole, 4,5-diamino-1-methyl-3-tert-butylpyrazole, 4,5-diamino-1-ethyl-3-methyl-pyrazole, 4,5-diamino-1-ethyl-3-(4'-methoxyphenyl)-pyrazole, 4,5-diamino-1-ethyl-3-hydroxymethyl-pyrazole, 4,5-diamino-3-hydroxymethyl-1-methyl-pyrazole, 4,5-diamino-3-hydroxymethyl-1-isopropyl-pyrazole, 4,5-diamino-3-methyl-1-isopropylpyrazole, and the addition salts thereof with an acid.

6. Composition according to any one of the preceding claims, **characterized in that** the heterocyclic oxidation base(s) represent(s) from 0.0005 to 12% by weight relative to the total weight of the dye composition.

7. Composition according to Claim 6, **characterized in that** the heterocyclic oxidation base(s) represent(s) from 0.005 to 6% by weight relative to the total weight of the dye composition.

8. Composition according to any one of the preceding claims, **characterized in that** the 3-aminopyridine derivative(s) of formula (I) is (are) chosen from:

- 4'-dimethylaminobenzene-1'-azo-1-methyl-3-pyridinium methosulphate of formula:

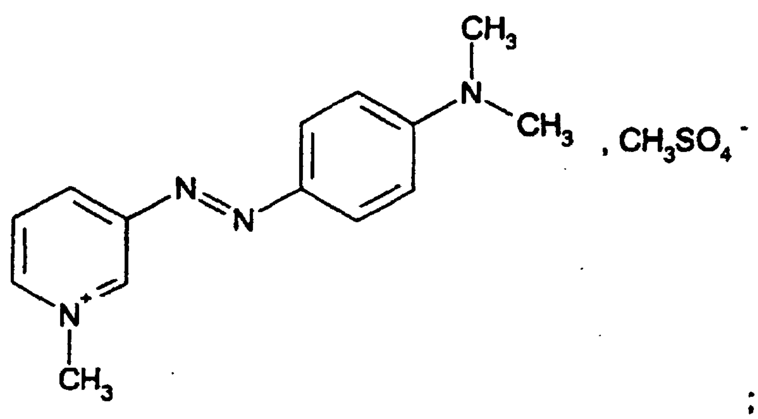

- 4'-bis(β-hydroxyethyl)aminobenzene-1'-azo-1-methyl-3-pyridinium methosulphate of formula:

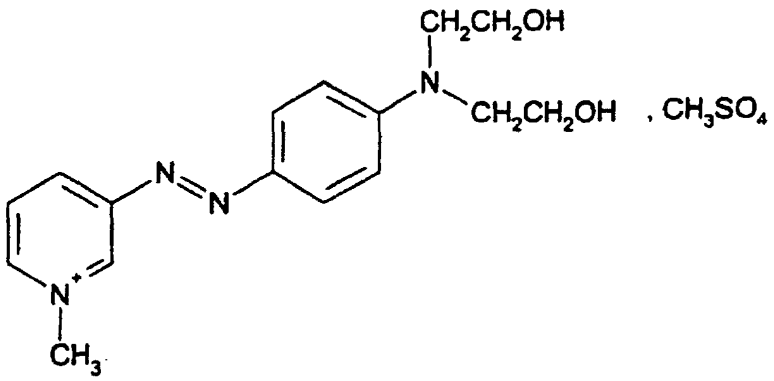

;

- 4'-amino-8'-hydroxynaphthalene-1'-azo-1-methyl-3-pyridinium methosulohate of formula:

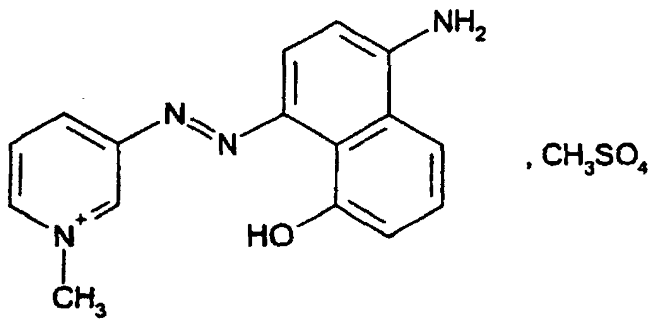

;

- 4'-dimethylamino-2'-nitrobenzene-1'-azo-1-methyl-3-pyridinium methosulphate of formula:

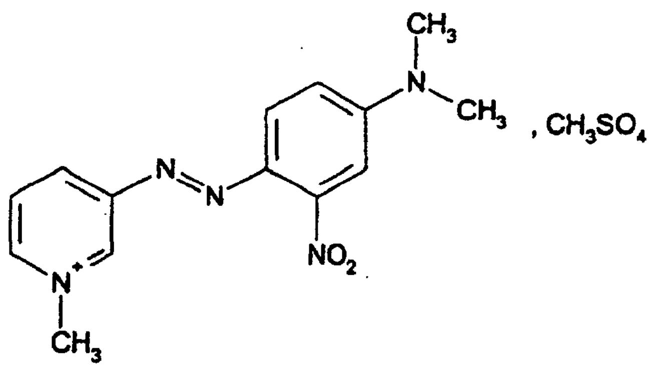

;

- 4'-dimethylaminobenzene-1'-azo-2,6-dimethyl-3-pyridinium methosulphate of formula:

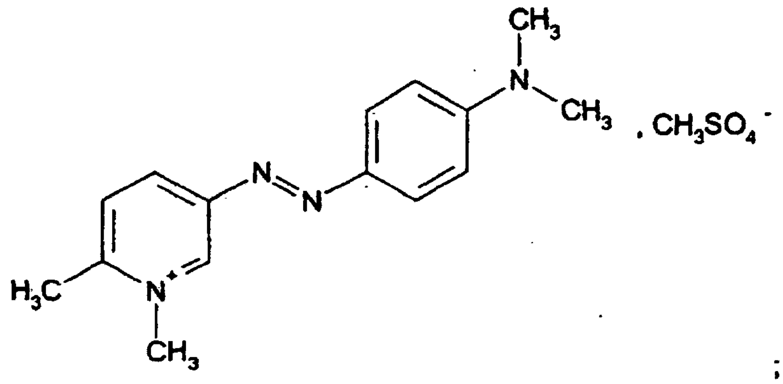

;

- 4'-aminobenzene-1'-azo-3-pyridine N-oxide of formula:

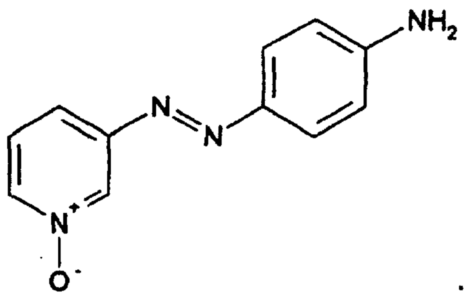

;

- 4'-dimethylaminobenzene-1'-azo-3-pyridine N-oxide of formula:

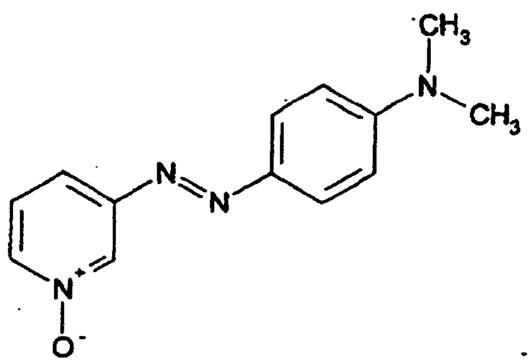

;

- 4'-N,N-bis(β-hydroxyethyl)aminobenzene-1'-azo-3-pyridine N-oxide of formula:

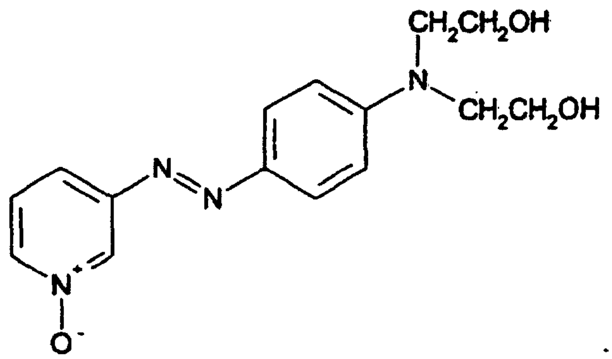

;

- 4'-dimethylamino-2'-methylbenzene-1'-azo-1-ethyl-3-pyridinium ethosulphate of formula:

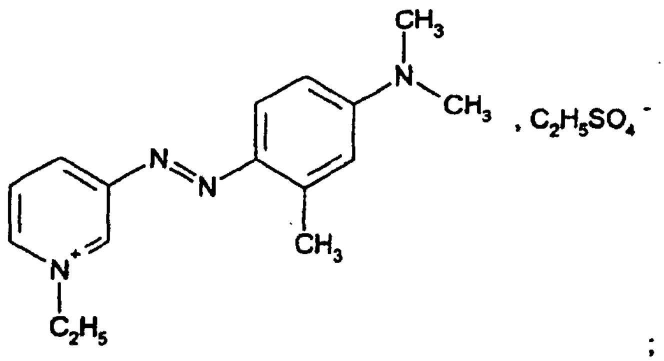

;

- 4'-dimethylamino-2'-methylbenzene-1'-azo-1-butyl-3-pyridinium bromide of formula:

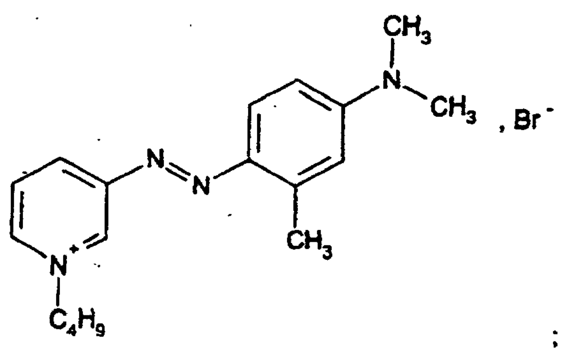

;

- 4'-dimethylamino-2'-chlorobenzene-1'-azo-1-methyl-3-pyridinium methosulphate of formula:

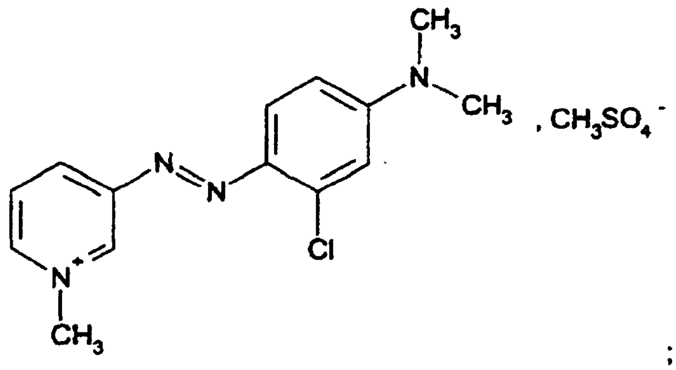

;

- 2',4'-diamino-5'-methylbenzene-1'-azo-1-methyl-3-pyridinium methosulphate of formula:

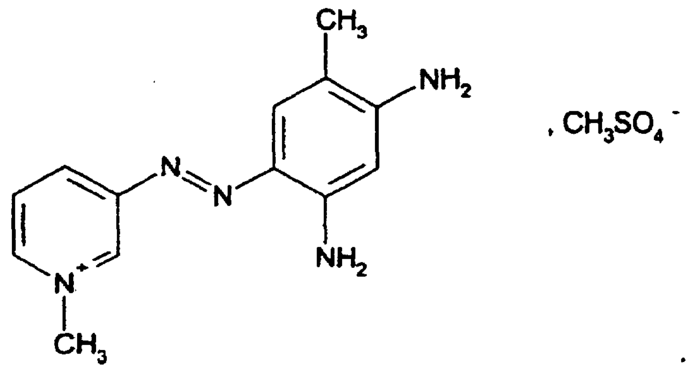

;

- 4'-phenylaminobenzene-1'-azo-1-methyl-3-pyridinium methosulphate of formula:

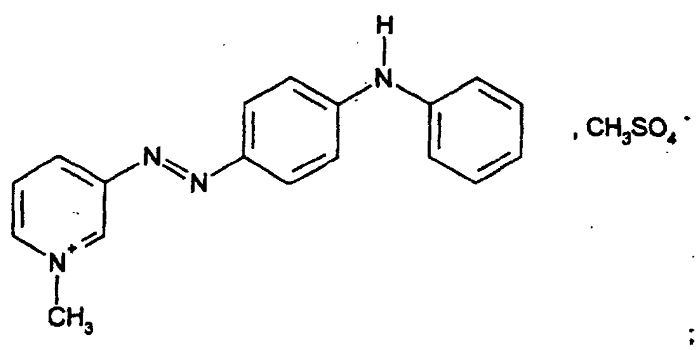

;

- 2'-acetylamino-4'-dimethylaminobenzene-1'-azo-1-ethyl-3-pyridinium ethosulphate of formula:

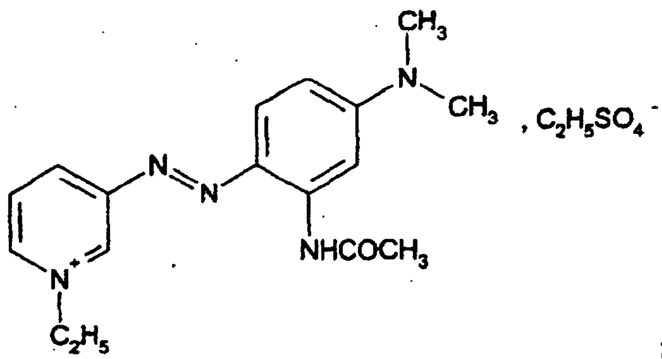

;

- 2',4'-diamino-5'-methoxybenzene-1'-azo-1-methyl-3-pyridinium methosulphate of formula:

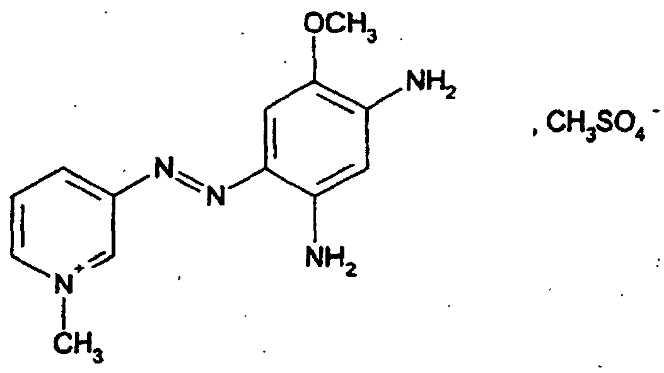

;

and

- 2'-amino-4'-dimethylaminobenzene-1'-azo-1-methyl-3-pyridinium methosulphate of formula:

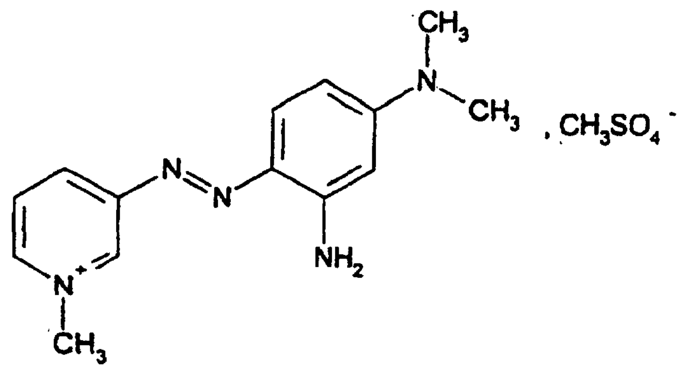

9. Composition according to any one of the preceding claims, **characterized in that** the 3-aminopyridine derivative(s) of formula (I) represent(s) from 0.001 to 10% by weight relative to the total weight of the dye composition.

10. Composition according to Claim 9, **characterized in that** the 3-aminopyridine derivative(s) of formula (I) represent(s) from 0.01 to 5% by weight relative to the total weight of the dye composition.

11. Composition according to any one of the preceding claims, **characterized in that** it contains one or more couplers and/or one or more benzenic oxidation bases and/or one or more direct dyes other than the 3-aminopyridine derivatives of formula (I) as defined in Claim 1.

**12.** Composition according to any one of the preceding claims, **characterized in that** the addition salts with an acid are chosen from the- hydrochlorides, hydrobromides, sulphates, tartrates, lactates and acetates.

**13.** Composition according to any one of the preceding claims, **characterized in that** the medium which is suitable for dyeing consists of water or of a mixture of water and at least one organic solvent.

**14.** Composition according to any one of the preceding claims, **characterized in that** it has a pH of between 3 and 12.

**15.** Process for dyeing keratin fibres, and in particular human keratin fibres such as the hair, **characterized in that** at least one dye composition as defined in any one of Claims 1 to 14 is applied to the said fibres, the colour being developed at acidic, neutral or alkaline pH with the aid of an oxidizing agent which is added to the dye composition only at the time of use, or which is present in an oxidizing composition that is applied simultaneously or sequentially.

**16.** Process according to Claim 15, **characterized in that** the oxidizing agent present in the oxidizing composition is chosen from hydrogen peroxide, urea peroxide, alkali metal bromates, persalts such as perborates, percarbonates and persulphates, peracids and enzymes.

**17.** Multi-compartment dyeing device or multicompartment dyeing "kit", a first compartment of which contains a dye composition as defined in any one of Claims 1 to 14 and a second compartment of which contains an oxidizing composition.